# EUROPEAN PATENT APPLICATION

(11) **EP 0 806 478 A2**
(43) Date of publication of application: **12.11.1997**
(21) Application number: 97107696.3
(22) Date of filing: 12.05.1997
(51) Int. Cl.: C12N 15/12, C12N 15/13, C07K 14/47, C07K 16/18, G01N 33/50, G01N 33/68

(54) **p53as Protein and antibody therefor**

(30) Priority: 10.05.1996 US 644456; 10.05.1996 US 644289; 10.05.1996 US 644291
(71) Applicant: HEALTH RESEARCH, INC., Buffalo New York 14263 (US)
(72) Inventor: Kulesz-Martin, Molly F., Buffalo, NY 14214 (US)
(74) Representative: Weber, Dieter, Dr.

(57) **Abstract**

The invention is a synthetic p53as protein for a mammal having at least 80% identity with p53 protein from that mammal and which has a same sequence specific binding on the cellular level as active p53 protein for that mammal except that said p53as sequence specific binding remains active in cellular environments in which p53 sequence specific binding is deactivated, said p53as differing from p53 within the final 50 carboxy terminal amino acids of p53, p53as containing a specific antigen site not present in p53, which gives rise to an antibody unique for p53as. The invention also includes a purified peptide designated p53as peptide, which peptide is present in the p53as protein and is within a unique carboxy terminal region which distinguishes p53as protein from p53 protein. An antibody is also included which specifically binds to the synthetic mammalian p53as protein and peptide and does not bind to a normal p53 from the same species wherein said antibody binds to an epitope present in a peptide unique to the p53as. The invention also includes a method for characterizing unknown cell products using the antibody and plasmids and vectors which generate the synthetic p53as.

SEQUENCE LISTING

(1) GENERAL INFORMATION:
(i) APPLICANT: Kulesz-Martin, Molly F.
(ii) TITLE OF INVENTION: p53as PROTEIN AND ANTIBODY
THEREFOR
(iii) NUMBER OF SEQUENCES: 16
(iv) CORRESPONDENCE ADDRESS:
(A) ADDRESSEE: Dunn & Associates
(B) STREET: P.O. Box 96
(C) CITY: Newfane
(D) STATE: New York
(E) COUNTRY: U.S.A.
(F) ZIP: 14108
(v) COMPUTER READABLE FORM:
(A) MEDIUM TYPE: Diskette - 3.50 inch, 1.44 Mb
Storage
(B) COMPUTER: Victor 300 SX/25 (IBM PC Compatible)
(C) OPERATING SYSTEM: MS-DOS Version 5.0
(D) SOFTWARE: Wordstar Professional Release 4
(vi) CURRENT APPLICATION DATA:
(A) APPLICATION NUMBER:
(B) FILING DATE:
(C) CLASSIFICATION:
(vii) PRIOR APPLICATION DATA:
(A) APPLICATION NUMBER: U.S. 08/644,456
(B) FILING DATE: May 10, 1996
(C) CLASSIFICATION: unknown
(viii) PRIOR APPLICATION DATA:
(A) APPLICATION NUMBER: U.S. 08/644,289
(B) FILING DATE: May 10, 1996
(C) CLASSIFICATION: Not yet known
(ix) PRIOR APPLICATION DATA:
(A) APPLICATION NUMBER: U.S. 08/644,291
(B) FILING DATE: May 10, 1996
(C) CLASSIFICATION: Not yet known
(x) ATTORNEY/AGENT INFORMATION:
(A) NAME: Dunn, Michael L.
(B) REGISTRATION NUMBER: 25,330
(C) REFERENCE/DOCKET NUMBER: RPP:135C EP
(xi) TELECOMMUNICATION INFORMATION:
(A) TELEPHONE: (716) 433-1661
(B) TELEFAX: (716) 433-1665

(2) INFORMATION FOR SEQ ID NO 1:
(i) SEQUENCE CHARACTERISTICS:
(A) LENGTH: 21
(B) TYPE: amino acid
(C) STRANDEDNESS: UNKNOWN
(D) TOPOLOGY: UNKNOWN
(ii) MOLECULE TYPE: peptide
(iii) HYPOTHETICAL: no
(iv) ANTI-SENSE: no
(v) FRAGMENT TYPE: n/a
(vi) ORIGINAL SOURCE:
(A) ORGANISM: n/a

(B) STRAIN: n/a
(C) INDIVIDUAL ISOLATE: n/a
(D) DEVELOPMENTAL STAGE: n/a
(E) HAPLOTYPE: n/a
(F) TISSUE TYPE: n/a
(G) CELL TYPE: n/a
(H) CELL LINE: n/a
(I) ORGANELLE: n/a
(vii) IMMEDIATE SOURCE:
(A) LIBRARY:
(B) CLONE:
(viii) POSITION IN GENOME:
(A) CHROMOSOME/SEGMENT: 17
(B) MAP POSITION: intron 10
(C) UNITS:
(ix) FEATURE: n/a
(A) NAME/KEY:
(B) LOCATION:
(C) IDENTIFICATION METHOD:
(D) OTHER INFORMATION:
(x) PUBLICATION INFORMATION:
(A) AUTHORS:
(B) TITLE:
(C) JOURNAL:
(D) VOLUME:
(E) ISSUE:
(F) PAGES:
(G) DATE:
(H) DOCUMENT NUMBER:
(I) FILING DATE:
(J) PUBLICATION DATE:
(K) RELEVANT RESIDUES IN SEQ ID NO:
(xi) SEQUENCE DESCRIPTION: SEQ ID NO: 1
Ser Leu Arg Pro Phe Lys Ala Leu Val Arg Glu Lys Gly His Arg Pro
1               5                   10                  15

Ser Ser His Ser Cys
            20

(3) INFORMATION FOR SEQ ID NO 2:
(i) SEQUENCE CHARACTERISTICS:
(A) LENGTH: 17
(B) TYPE: amino acids
(C) STRANDEDNESS: n/a
(D) TOPOLOGY: n/a
(ii) MOLECULE TYPE: peptide
(iii) HYPOTHETICAL: no
(iv) ANTI-SENSE: no
(v) FRAGMENT TYPE: n/a
(vi) ORIGINAL SOURCE:
(A) ORGANISM: mouse
(B) STRAIN: n/a
(C) INDIVIDUAL ISOLATE: n/a
(D) DEVELOPMENTAL STAGE: n/a
(E) HAPLOTYPE: n/a

(F) TISSUE TYPE: n/a
(G) CELL TYPE: n/a
(H) CELL LINE: n/a
(I) ORGANELLE: n/a
(vii) IMMEDIATE SOURCE: sequenced from cDNA clone from
mouse epidermal cell RNA, Genbank Accession #M13874
(A) LIBRARY: plasmid p6.3
(B) CLONE:
(viii) POSITION IN GENOME:
(A) CHROMOSOME/SEGMENT: 11
(B) MAP POSITION: p53 gene
(C) UNITS:
(ix) FEATURE: n/a
(A) NAME/KEY:
(B) LOCATION:
(C) IDENTIFICATION METHOD:
(D) OTHER INFORMATION:
(x) PUBLICATION INFORMATION:
(A) AUTHORS: Kulesz-Martin et al.
(B) TITLE: Endogenous p53 Protein Generated From
Wild Type Alternatively Spliced P53 RNA in
Mouse
(C) JOURNAL: Mol. Cell. Biol.
(D) VOLUME: 14
(E) ISSUE: 3
(F) PAGES: 1698-1708
(G) DATE: March, 1994
(A) AUTHORS: Han, K.A. and Kulesz-Martin, M.F.
(B) TITLE: Alternatively Spliced p53 RNA in
Transformed and Normal Cells of Different
Tissue Types
(C) JOURNAL: Nucleic Acids Res.
(D) VOLUME: 20
(E) ISSUE: 8
(F) PAGES: 1979-1981
(G) DATE: 1992
(A) AUTHORS: Arai, N. et al.
(B) TITLE: Immunologically Distinct p53 Molecules
Generated by Alternative Splicing
(C) JOURNAL: Mol. and Cell. Biol.
(D) VOLUME: 6
(E) ISSUE:
(F) PAGES: 3232-3239
(G) DATE: 1986
(H) DOCUMENT NUMBER:
(I) FILING DATE:
(J) PUBLICATION DATE:
(K) RELEVANT RESIDUES IN SEQ ID NO:
(xi) SEQUENCE DESCRIPTION: SEQ ID NO: 2
Leu Gln Pro Arg Ala Phe Gln Ala Leu Ile Lys Glu Glu Ser Pro Asn
1               5                   10                  15

Cys

(4) INFORMATION FOR SEQ ID NO: 3:

(i) SEQUENCE CHARACTERISTICS:
(A) LENGTH: 33
(B) TYPE: Nucleic Acids
(C) STRANDEDNESS: Unknown
(D) TOPOLOGY: Unknown
(ii) MOLECULE TYPE: Oligonucleotide
(iii) HYPOTHETICAL:
(iv) ANTI-SENSE:
(v) FRAGMENT TYPE:
(vi) ORIGINAL SOURCE:
(A) ORGANISM: Human
(B) STRAIN:
(C) INDIVIDUAL ISOLATE:
(D) DEVELOPMENTAL STAGE:
(E) HAPLOTYPE:
(F) TISSUE TYPE:
(G) CELL TYPE:
(H) CELL LINE:
(I) ORGANELLE:
(vii) IMMEDIATE SOURCE: Genbank Accession #X54156, Locus
HUM P53G
(A) LIBRARY:
(B) CLONE
(viii) POSITION IN GENOME:
(A) CHROMOSOME/SEGMENT: 17
(B) MAP POSITION: p53 gene 17593-17613
(C) UNITS:
(ix) FEATURE:
(A) NAME/KEY:
(B) LOCATION:
(C) IDENTIFICATION METHOD:
(D) OTHER INFORMATION:
(x) PUBLICATION INFORMATION:
(A) AUTHORS:
(B) TITLE:
(C) JOURNAL:
(D) VOLUME:
(E) ISSUE:
(F) PAGES:
(G) DATE:
(H) DOCUMENT NUMBER:
(I) FILING DATE:
(J) PUBLICATION DATE:
(K) RELEVANT RESIDUES IN SEQ ID NO:
(xi) SEQUENCE DESCRIPTION: SEQ ID NO: 3
ATCGAAGCTT GAGATGTTCC GAGAGAGCTG AAT 33

(5) INFORMATION FOR SEQ ID NO: 4:
(i) SEQUENCE CHARACTERISTICS:
(A) LENGTH: 31
(B) TYPE: Nucleic acids
(C) STRANDEDNESS: unknown
(D) TOPOLOGY: unknown
(ii) MOLECULE TYPE: oligonucleotide

(iii) HYPOTHETICAL:
(iv) ANTI-SENSE:
(v) FRAGMENT TYPE:
(vi) ORIGINAL SOURCE:
(A) ORGANISM: Human
(B) STRAIN:
(C) INDIVIDUAL ISOLATE:
(D) DEVELOPMENTAL STAGE:
(E) HAPLOTYPE:
(F) TISSUE TYPE:
(G) CELL TYPE:
(H) CELL LINE:
(I) ORGANELLE:
(vii) IMMEDIATE SOURCE: Genbank Accession #54156,
Locus HUMP53G
(A) LIBRARY:
(B) CLONE
(viii) POSITION IN GENOME:
(A) CHROMOSOME/SEGMENT: 17
(B) MAP POSITION: p53 gene 18774-18794
(C) UNITS:
(ix) FEATURE:
(A) NAME/KEY:
(B) LOCATION:
(C) IDENTIFICATION METHOD:
(D) OTHER INFORMATION:
(x) PUBLICATION INFORMATION:
(A) AUTHORS:
(B) TITLE:
(C) JOURNAL:
(D) VOLUME:
(E) ISSUE:
(F) PAGES:
(G) DATE:
(H) DOCUMENT NUMBER:
(I) FILING DATE:
(J) PUBLICATION DATE:
(K) RELEVANT RESIDUES IN SEQ ID NO:
(xi) SEQUENCE DESCRIPTION: SEQ ID NO: 4
ATCGTCTAGA GCTTCTGACG CACACCTATT G 31

(6) INFORMATION FOR SEQ ID NO: 5:
(i) SEQUENCE CHARACTERISTICS:
(A) LENGTH: 20
(B) TYPE: Amino Acids
(C) STRANDEDNESS: unknown
(D) TOPOLOGY: unknown
(ii) MOLECULE TYPE: Peptide
(iii) HYPOTHETICAL: deduced from intron 10 sequences p53
gene
(iv) ANTI-SENSE:
(v) FRAGMENT TYPE:
(vi) ORIGINAL SOURCE:
(A) ORGANISM: Human

(B) STRAIN:
(C) INDIVIDUAL ISOLATE:
(D) DEVELOPMENTAL STAGE:
(E) HAPLOTYPE:
(F) TISSUE TYPE:
(G) CELL TYPE:
(H) CELL LINE:
(I) ORGANELLE:
(vii) IMMEDIATE SOURCE:
(A) LIBRARY: deduced translation from nucleotides in
Genbank nucleic acid database accession #54156,
Locus HSP53G
(B) CLONE:
(viii) POSITION IN GENOME:
(A) CHROMOSOME/SEGMENT: 17
(B) MAP POSITION: p53 gene, at 18530 to 18589
(C) UNITS:
(ix) FEATURE: n/a
(A) NAME/KEY:
(B) LOCATION:
(C) IDENTIFICATION METHOD:
(D) OTHER INFORMATION:
(x) PUBLICATION INFORMATION:
(A) AUTHORS:
(B) TITLE:
(C) JOURNAL:
(D) VOLUME:
(E) ISSUE:
(F) PAGES:
(G) DATE:
(H) DOCUMENT NUMBER:
(I) FILING DATE:
(J) PUBLICATION DATE:
(K) RELEVANT RESIDUES IN SEQ ID NO.:
(xi) SEQUENCE DESCRIPTION: SEQ ID NO: 5
Arg Glu Lys Gly His Arg Pro Ser His Ser Cys Asp Val Ile Ser Pro
1               5                   10                  15

Pro Cys Phe Cys
            20

(7) INFORMATION FOR SEQ ID NO: 6:
(i) SEQUENCE CHARACTERISTICS:
(A) LENGTH: 16
(B) TYPE: Amino Acids
(C) STRANDEDNESS: unknown
(D) TOPOLOGY: unknown
(ii) MOLECULE TYPE: peptide
(iii) HYPOTHETICAL:
(iv) ANTI-SENSE:
(v) FRAGMENT TYPE:
(vi) ORIGINAL SOURCE: mouse glucocorticoid receptor sequence
(A) ORGANISM:
(B) STRAIN:
(C) INDIVIDUAL ISOLATE:

(D) DEVELOPMENTAL STAGE:
(E) HAPLOTYPE:
(F) TISSUE TYPE:
(G) CELL TYPE:
(H) CELL LINE:
(I) ORGANELLE:
(vii) IMMEDIATE SOURCE:
(A) LIBRARY:
(B) CLONE
(viii) POSITION IN GENOME:
(A) CHROMOSOME/SEGMENT:
(B) MAP POSITION:
(C) UNITS:
(ix) FEATURE:
(A) NAME/KEY:
(B) LOCATION:
(C) IDENTIFICATION METHOD:
(D) OTHER INFORMATION:
(x) PUBLICATION INFORMATION:
(A) AUTHORS:
(B) TITLE:
(C) JOURNAL:
(D) VOLUME:
(E) ISSUE:
(F) PAGES:
(G) DATE:
(H) DOCUMENT NUMBER:
(I) FILING DATE:
(J) PUBLICATION DATE:
(K) RELEVANT RESIDUES IN SEQ ID NO:
(xi) SEQUENCE DESCRIPTION: SEQ ID NO: 6
Gly Arg Asn Asp Cys Ile Ile Asp Lys Ile Arg Arg Lys Asn Cys Asp
1               5                   10                  15

(8) INFORMATION FOR SEQ ID NO: 7:
(i) SEQUENCE CHARACTERISTICS:
(A) LENGTH: 38
(B) TYPE: Nucleic Acid
(C) STRANDEDNESS: unknown
(D) TOPOLOGY: unknown
(ii) MOLECULE TYPE:
(iii) HYPOTHETICAL: No
(iv) ANTI-SENSE: No
(v) FRAGMENT TYPE:
(vi) ORIGINAL SOURCE: Murine
(A) ORGANISM: Balb/c
(B) STRAIN:
(C) INDIVIDUAL ISOLATE:
(D) DEVELOPMENTAL STAGE:
(E) HAPLOTYPE:
(F) TISSUE TYPE:
(G) CELL TYPE:
(H) CELL LINE:
(I) ORGANELLE:
(vii) IMMEDIATE SOURCE:

(A) LIBRARY:
(B) CLONE
(viii) POSITION IN GENOME:
(A) CHROMOSOME/SEGMENT:
(B) MAP POSITION:
(C) UNITS:
(ix) FEATURE:
(A) NAME/KEY:
(B) LOCATION:
(C) IDENTIFICATION METHOD:
(D) OTHER INFORMATION:
(x) PUBLICATION INFORMATION:
(A) AUTHORS: K. Han, M. Kulesz-Martin
(B) TITLE: ALTERNATIVELY SPLICED p53 RNA IN
TRANSFORMED AND NORMAL CELLS OF
DIFFERENT TISSUE TYPES
(C) JOURNAL: Nuc. Acids Res.
(D) VOLUME: 20
(E) ISSUE: 8
(F) PAGES: 1979-81
(G) DATE: 1992
(H) DOCUMENT NUMBER:
(I) FILING DATE:
(J) PUBLICATION DATE:
(K) RELEVANT RESIDUES IN SEQ ID NO:
(xi) SEQUENCE DESCRIPTION: SEQ ID NO: 7
AGTCAGGCCT TAGAGTTAAA GGATGCCCAT GCTACAGA 38

(9) INFORMATION FOR SEQ ID NO: 8:
(i) SEQUENCE CHARACTERISTICS:
(A) LENGTH: 28
(B) TYPE: Nucleic Acid
(C) STRANDEDNESS: unknown
(D) TOPOLOGY: unknown
(ii) MOLECULE TYPE:
(iii) HYPOTHETICAL: No
(iv) ANTI-SENSE: No
(v) FRAGMENT TYPE:
(vi) ORIGINAL SOURCE: Murine
(A) ORGANISM: Balb/c
(B) STRAIN:
(C) INDIVIDUAL ISOLATE:
(D) DEVELOPMENTAL STAGE:
(E) HAPLOTYPE:
(F) TISSUE TYPE:
(G) CELL TYPE:
(H) CELL LINE:
(I) ORGANELLE:
(vii) IMMEDIATE SOURCE:
(A) LIBRARY:
(B) CLONE
(viii) POSITION IN GENOME:
(A) CHROMOSOME/SEGMENT:
(B) MAP POSITION:
(C) UNITS:

(ix) FEATURE:
(A) NAME/KEY:
(B) LOCATION:
(C) IDENTIFICATION METHOD:
(D) OTHER INFORMATION:
(x) PUBLICATION INFORMATION:
(A) AUTHORS:
(B) TITLE:
(C) JOURNAL:
(D) VOLUME:
(E) ISSUE:
(F) PAGES:
(G) DATE:
(H) DOCUMENT NUMBER:
(I) FILING DATE:
(J) PUBLICATION DATE:
(K) RELEVANT RESIDUES IN SEQ ID NO:
(xi) SEQUENCE DESCRIPTION: SEQ ID NO: 8
AGTCGAATTC ATTGGGACCA TCCTGGCT 28

(10) INFORMATION FOR SEQ ID NO: 9:
(i) SEQUENCE CHARACTERISTICS:
(A) LENGTH: 30
(B) TYPE: Nucleic Acid
(C) STRANDEDNESS: unknown
(D) TOPOLOGY: unknown
(ii) MOLECULE TYPE:
(iii) HYPOTHETICAL: No
(iv) ANTI-SENSE: No
(v) FRAGMENT TYPE:
(vi) ORIGINAL SOURCE: Murine
(A) ORGANISM: Balb/c
(B) STRAIN:
(C) INDIVIDUAL ISOLATE:
(D) DEVELOPMENTAL STAGE:
(E) HAPLOTYPE:
(F) TISSUE TYPE:
(G) CELL TYPE:
(H) CELL LINE:
(I) ORGANELLE:
(vii) IMMEDIATE SOURCE:
(A) LIBRARY:
(B) CLONE
(viii) POSITION IN GENOME:
(A) CHROMOSOME/SEGMENT:
(B) MAP POSITION:
(C) UNITS:
(ix) FEATURE:
(A) NAME/KEY:
(B) LOCATION:
(C) IDENTIFICATION METHOD:
(D) OTHER INFORMATION:
(x) PUBLICATION INFORMATION:
(A) AUTHORS:

(B) TITLE:
(C) JOURNAL:
(D) VOLUME:
(E) ISSUE:
(F) PAGES:
(G) DATE:
(H) DOCUMENT NUMBER:
(I) FILING DATE:
(J) PUBLICATION DATE:
(K) RELEVANT RESIDUES IN SEQ ID NO:
(xi) SEQUENCE DESCRIPTION: SEQ ID NO: 9
AGTCGGATCC TGGAGTGAGC CCTGCTGTCT 30

(11) INFORMATION FOR SEQ ID NO: 10:
(i) SEQUENCE CHARACTERISTICS:
(A) LENGTH: 10
(B) TYPE: Nucleic Acid
(C) STRANDEDNESS: unknown
(D) TOPOLOGY: unknown
(ii) MOLECULE TYPE:
(iii) HYPOTHETICAL: No
(iv) ANTI-SENSE: No
(v) FRAGMENT TYPE:
(vi) ORIGINAL SOURCE:
(A) ORGANISM:
(B) STRAIN:
(C) INDIVIDUAL ISOLATE:
(D) DEVELOPMENTAL STAGE:
(E) HAPLOTYPE:
(F) TISSUE TYPE:
(G) CELL TYPE:
(H) CELL LINE:
(I) ORGANELLE:
(vii) IMMEDIATE SOURCE:
(A) LIBRARY:
(B) CLONE
(viii) POSITION IN GENOME:
(A) CHROMOSOME/SEGMENT:
(B) MAP POSITION:
(C) UNITS:
(ix) FEATURE:
(A) NAME/KEY:
(B) LOCATION:
(C) IDENTIFICATION METHOD:
(D) OTHER INFORMATION:
(x) PUBLICATION INFORMATION:
(A) AUTHORS: El - Diery et al.
(B) TITLE: DEFINITION OF A CONCENCUS BINDING
SITE FOR p53
(C) JOURNAL: Nature Genetics
(D) VOLUME: 1
(E) ISSUE:
(F) PAGES: 45-49
(G) DATE: April, 1992
(H) DOCUMENT NUMBER:

(I) FILING DATE:
(J) PUBLICATION DATE:
(K) RELEVANT RESIDUES IN SEQ ID NO:
(xi) SEQUENCE DESCRIPTION: SEQ ID NO: 10
AGGCWWGCCT 10

(12) INFORMATION FOR SEQ ID NO: 11:
(i) SEQUENCE CHARACTERISTICS:
(A) LENGTH: 10
(B) TYPE: Nucleic Acid
(C) STRANDEDNESS: unknown
(D) TOPOLOGY: unknown
(ii) MOLECULE TYPE:
(iii) HYPOTHETICAL: No
(iv) ANTI-SENSE: No
(v) FRAGMENT TYPE:
(vi) ORIGINAL SOURCE:
(A) ORGANISM:
(B) STRAIN:
(C) INDIVIDUAL ISOLATE:
(D) DEVELOPMENTAL STAGE:
(E) HAPLOTYPE:
(F) TISSUE TYPE:
(G) CELL TYPE:
(H) CELL LINE:
(I) ORGANELLE:
(vii) IMMEDIATE SOURCE:
(A) LIBRARY:
(B) CLONE
(viii) POSITION IN GENOME:
(A) CHROMOSOME/SEGMENT:
(B) MAP POSITION:
(C) UNITS:
(ix) FEATURE:
(A) NAME/KEY:
(B) LOCATION:
(C) IDENTIFICATION METHOD:
(D) OTHER INFORMATION:
(x) PUBLICATION INFORMATION:
(A) AUTHORS: El - Diery et al.
(B) TITLE: N/A
(C) JOURNAL: Nature
(D) VOLUME: 358
(E) ISSUE: N/A
(F) PAGES: 83-86
(G) DATE: 1992
(H) DOCUMENT NUMBER:
(I) FILING DATE:
(J) PUBLICATION DATE:
(K) RELEVANT RESIDUES IN SEQ ID NO:
(xi) SEQUENCE DESCRIPTION: SEQ ID NO: 11
AGGCATGCCT 10

(13) INFORMATION FOR SEQ ID NO: 12:
(i) SEQUENCE CHARACTERISTICS:

(A) LENGTH: 10
(B) TYPE: Nucleic Acid
(C) STRANDEDNESS: unknown
(D) TOPOLOGY: unknown
(ii) MOLECULE TYPE:
(iii) HYPOTHETICAL: No
(iv) ANTI-SENSE: No
(v) FRAGMENT TYPE:
(vi) ORIGINAL SOURCE:
(A) ORGANISM:
(B) STRAIN:
(C) INDIVIDUAL ISOLATE:
(D) DEVELOPMENTAL STAGE:
(E) HAPLOTYPE:
(F) TISSUE TYPE:
(G) CELL TYPE:
(H) CELL LINE:
(I) ORGANELLE:
(vii) IMMEDIATE SOURCE:
(A) LIBRARY:
(B) CLONE
(viii) POSITION IN GENOME:
(A) CHROMOSOME/SEGMENT:
(B) MAP POSITION:
(C) UNITS:
(ix) FEATURE:
(A) NAME/KEY:
(B) LOCATION:
(C) IDENTIFICATION METHOD:
(D) OTHER INFORMATION:
(x) PUBLICATION INFORMATION:
(A) AUTHORS: El - Diery et al.
(B) TITLE: N/A
(C) JOURNAL: Nature
(D) VOLUME: 358
(E) ISSUE: N/A
(F) PAGES: 83-86
(G) DATE: 1992
(H) DOCUMENT NUMBER:
(I) FILING DATE:
(J) PUBLICATION DATE:
(K) RELEVANT RESIDUES IN SEQ ID NO:
(xi) SEQUENCE DESCRIPTION: SEQ ID NO: 12
AGGAATTCCT 10

(14) INFORMATION FOR SEQ ID NO: 13:
(i) SEQUENCE CHARACTERISTICS:
(A) LENGTH: 50
(B) TYPE: Nucleic Acid
(C) STRANDEDNESS: unknown
(D) TOPOLOGY: unknown
(ii) MOLECULE TYPE:
(iii) HYPOTHETICAL: No
(iv) ANTI-SENSE: No
(v) FRAGMENT TYPE:

(vi) ORIGINAL SOURCE:
(A) ORGANISM:
(B) STRAIN:
(C) INDIVIDUAL ISOLATE:
(D) DEVELOPMENTAL STAGE:
(E) HAPLOTYPE:
(F) TISSUE TYPE:
(G) CELL TYPE:
(H) CELL LINE:
(I) ORGANELLE:
(vii) IMMEDIATE SOURCE:
(A) LIBRARY:
(B) CLONE
(viii) POSITION IN GENOME:
(A) CHROMOSOME/SEGMENT:
(B) MAP POSITION:
(C) UNITS:
(ix) FEATURE:
(A) NAME/KEY:
(B) LOCATION:
(C) IDENTIFICATION METHOD:
(D) OTHER INFORMATION:
(x) PUBLICATION INFORMATION:
(A) AUTHORS: Zambetti et al.
(B) TITLE: WILD-TYPE p53 MEDIATES POSITIVE
REGULATION OF GENE EXPRESSION
THROUGH A SPECIFIC DNA SEQUENCE
ELEMENT
(C) JOURNAL: Genes & Development
(D) VOLUME: 6
(E) ISSUE: N/A
(F) PAGES: 1143-52
(G) DATE: 1992
(H) DOCUMENT NUMBER:
(I) FILING DATE:
(J) PUBLICATION DATE:
(K) RELEVANT RESIDUES IN SEQ ID NO:
(xi) SEQUENCE DESCRIPTION: SEQ ID NO: 13
TGGCAAGCCT ATGACATGGC CGGGGCCTGC CTCTCTCTGC CTCTGACCCT 50

(15) INFORMATION FOR SEQ ID NO: 14:
(i) SEQUENCE CHARACTERISTICS:
(A) LENGTH: 30
(B) TYPE: Nucleic Acid
(C) STRANDEDNESS: unknown
(D) TOPOLOGY: unknown
(ii) MOLECULE TYPE:
(iii) HYPOTHETICAL: No
(iv) ANTI-SENSE: No
(v) FRAGMENT TYPE:
(vi) ORIGINAL SOURCE:
(A) ORGANISM:
(B) STRAIN:
(C) INDIVIDUAL ISOLATE:
(D) DEVELOPMENTAL STAGE:

(E) HAPLOTYPE:
(F) TISSUE TYPE:
(G) CELL TYPE:
(H) CELL LINE:
(I) ORGANELLE:
(vii) IMMEDIATE SOURCE:
(A) LIBRARY:
(B) CLONE
(viii) POSITION IN GENOME:
(A) CHROMOSOME/SEGMENT:
(B) MAP POSITION:
(C) UNITS:
(ix) FEATURE:
(A) NAME/KEY:
(B) LOCATION:
(C) IDENTIFICATION METHOD:
(D) OTHER INFORMATION:
(x) PUBLICATION INFORMATION:
(A) AUTHORS: Foord et al.
(B) TITLE: ISOLATION AND CHARACTERIZATION OF
DNA SEQUENCES THAT ARE SPECIFICALLY
BOUND BY WILD-TYPE p53 PROTEIN
(C) JOURNAL: Mol. Cell. Biol.
(D) VOLUME: 13
(E) ISSUE: 3
(F) PAGES: 1378-84
(G) DATE: 1993
(H) DOCUMENT NUMBER:
(I) FILING DATE:
(J) PUBLICATION DATE:
(K) RELEVANT RESIDUES IN SEQ ID NO:
(xi) SEQUENCE DESCRIPTION: SEQ ID NO: 14
GACACTGGTC ACACTTGGCT GCTTAGGAAT 30

(16) INFORMATION FOR SEQ ID NO: 15
(i) SEQUENCE CHARACTERISTICS:
(A) LENGTH: 27
(B) TYPE: Amino Acid
(C) STRANDEDNESS: unknown
(D) TOPOLOGY: unknown
(ii) MOLECULE TYPE: Peptide
(iii) HYPOTHETICAL: No
(iv) ANTI-SENSE: No
(v) FRAGMENT TYPE:
(vi) ORIGINAL SOURCE:
(A) ORGANISM:
(B) STRAIN:
(C) INDIVIDUAL ISOLATE:
(D) DEVELOPMENTAL STAGE:
(E) HAPLOTYPE:
(F) TISSUE TYPE:
(G) CELL TYPE:
(H) CELL LINE:
(I) ORGANELLE:
(vii) IMMEDIATE SOURCE:

(A) LIBRARY:
(B) CLONE
(viii) POSITION IN GENOME:
(A) CHROMOSOME/SEGMENT:
(B) MAP POSITION:
(C) UNITS:
(ix) FEATURE:
(A) NAME/KEY:
(B) LOCATION:
(C) IDENTIFICATION METHOD:
(D) OTHER INFORMATION:
(x) PUBLICATION INFORMATION:
(A) AUTHORS:
(B) TITLE:
(C) JOURNAL:
(D) VOLUME:
(E) ISSUE:
(F) PAGES:
(G) DATE:
(H) DOCUMENT NUMBER:
(I) FILING DATE:
(J) PUBLICATION DATE:
(K) RELEVANT RESIDUES IN SEQ ID NO:
(xi) SEQUENCE DESCRIPTION: SEQ ID NO: 15
Ser Tyr Leu Lys Thr Lys Lys Gly Gln Ser Thr Ser Arg His Lys Lys
1               5                   10                  15

Thr Met Val Lys Lys Val Gly Pro Asp Ser Asp
            20                  25

(17) INFORMATION FOR SEQ ID NO: 16
(i) SEQUENCE CHARACTERISTICS:
(A) LENGTH: 18
(B) TYPE: Amino Acid
(C) STRANDEDNESS: unknown
(D) TOPOLOGY: unknown
(ii) MOLECULE TYPE: Peptide
(iii) HYPOTHETICAL: No
(iv) ANTI-SENSE: No
(v) FRAGMENT TYPE:
(vi) ORIGINAL SOURCE:
(A) ORGANISM:
(B) STRAIN:
(C) INDIVIDUAL ISOLATE:
(D) DEVELOPMENTAL STAGE:
(E) HAPLOTYPE:
(F) TISSUE TYPE:
(G) CELL TYPE:
(H) CELL LINE:
(I) ORGANELLE:
(vii) IMMEDIATE SOURCE:
(A) LIBRARY:
(B) CLONE
(viii) POSITION IN GENOME:
(A) CHROMOSOME/SEGMENT:

(B) MAP POSITION:
(C) UNITS:
(ix) FEATURE:
(A) NAME/KEY:
(B) LOCATION:
(C) IDENTIFICATION METHOD:
(D) OTHER INFORMATION:
(x) PUBLICATION INFORMATION:
(A) AUTHORS:
(B) TITLE:
(C) JOURNAL:
(D) VOLUME:
(E) ISSUE:
(F) PAGES:
(G) DATE:
(H) DOCUMENT NUMBER:
(I) FILING DATE:
(J) PUBLICATION DATE:
(K) RELEVANT RESIDUES IN SEQ ID NO:
(xi) SEQUENCE DESCRIPTION: SEQ ID NO: 16
Ser Leu Gln Pro Arg Ala Phe Gln Ala Leu Ile Lys Glu Glu Ser Pro
                5                   10                  15

Asn Cys

## Description

### Background of the Invention

This invention relates to p53 protein and variations thereof, and more particularly relates to antibodies to such variations, vectors containing coding sequences for such variations and uses for such variations, antibodies and vectors.

p53 protein is a protein encoded by p53 gene. The p53 gene has a size of from 10 to 20Kb, usually has eleven exons, is believed to occur in all vertebrates and such p53 genes have at least five significant homologous domains all the way from Xenopus up through primates. Structural aspects of the p53 protein in relation to gene evolution, Soussi et al., Oncogene, (1990), 5, 945-952; The p53 Tumor Suppressor Protein; Meeting Review, Prives et al, Genes and Development, (1993) 7:529-534. In mice and humans, a single copy of a functional p53 gene is found on haploid genomes where it is located on chromosome 11.

p53 protein contains a negative regulatory domain for p53 sequence specific DNA binding which is found within the last 50 amino acids at the p53 C terminus. The negative regulatory domain of p53 negates p53 sequence specific binding in certain cellular environments which in turn causes p53 to lose activity.

The p53 gene which encodes for p53 protein is defective in over half of all human cancers. It is furthermore significant because introduction of a normal p53 gene into a variety of cancer cells arrests their growth. Thus, defects in the p53 gene product (that is, the p53 protein) are common in many cancers and, if corrected, could inhibit cancer cell growth. In many human cancers, the p53 protein is inactive because of mutation of the p53 gene. Replacement of a single amino acid can be sufficient to change the normal folding of the p53 protein, making it inactive as a growth control gene. In certain cells, the folding of a mutant p53 protein can be stabilized in the normal conformation by binding to cellular factors, suggesting that it may be possible to create peptides which bind to p53 protein and cause it to be maintained in the normal conformation (conformations are forms of a protein created due to folding; conformations can change without (or with) changes in amino acid sequence). The normal conformation has the tumor suppressor effect. Cells expressing primarily mutant p53 conformation give rise to aggressive tumors at high frequency while cells which primarily express p53 protein in a normal conformation give rise to slow-growing tumors at low frequency.

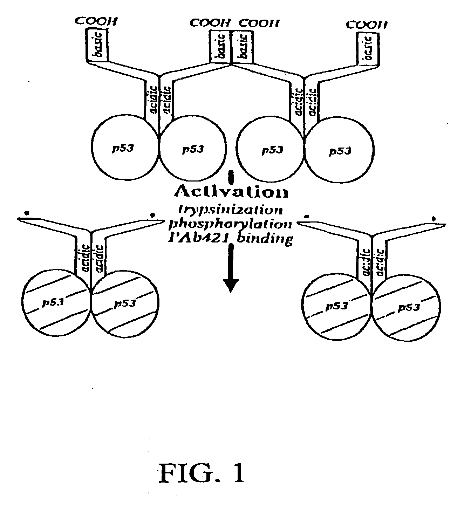

To date, many studies of p53 protein and its function have relied upon a specific (PAb421) antibody thereto. Most p53 proteins studied using *in vitro* (cell-free) assays of binding to DNA or modulation of transcription have used a p53 protein purified using PAb421, and thus excluding other proteins. While p53 binding detectable to date is sequence specific, it is low in efficiency. A model has been proposed for activation of p53 protein for binding to DNA by modifications at the carboxyl terminus of p53, Hupp et al. (1992) "Regulation of the specific DNA binding function of p53", Cell 71, 875-886, as shown in Figure 1. Modifications include proteolysis (loss of carboxyl terminal amino acids), phosphorylation of serine in this region or binding of PAb421 antibody within this region.

It has been shown that p53as RNA exists in normal mouse cells and tissues and in tumor cells (Han et al. (b) (1992), "Alternatively spliced p53 RNA in transformed and normal cells of different tissue types", Nucleic Acids Res., 20(8), 1979-1981.

It has been demonstrated previously that a wild type alternatively spliced p53 (p53as, for alternative splice) RNA exists in cultured cells and normal tissues of mice at approximately 30% of the major p53 RNA form (Han and Kulesz-Martin, Nucleic Acid Res., 20:1979-81, 1992). The predicted protein encoded by the p53as transcript differs from p53 protein in 17 C-terminal amino acids and is truncated by 9 amino acids due to alternative splicing of intron 10 of the wild type p53 gene. Using antibody to the 17 C-terminal amino acids to detect p53as protein, the following points have been demonstrated. Natural p53as protein is an alternatively spliced product of the wild type p53 gene. First detected in mouse epidermal cells, it is present in non-transformed and malignant cells. Like its major counterpart, p53 protein, it is located in the nucleus. However, while p53 antigen activity is primarily found in cells at the G1 stage of the cell cycle and is thought to play a role in G1 arrest in cells following treatment with DNA damaging agents, p53as is found in cells preferentially distributed in the G2 phase of the cell cycle and in a "tail" of cells with >G2 DNA content. These properties of p53as protein are suggestive of cellular functions distinct from the major p53 protein. The well established ability of the p53 protein to oligomerize and the finding of co-expression of p53as antigen activity with p53 in cells suggested potential for cooperation with p53 in its functions related to cell cycle control.

The finding of naturally occurring p53as protein in mouse tumor cells and antibodies for its detection has applications in basic research on cell growth and differentiation. The development of a homologous protein for use in human cells has applications in the diagnosis, prognosis and design of treatment strategy in human diseases of growth and differentiation such as cancer. The association with G2 suggests a functional role in G2 arrest and potential for gene therapy using the p53as coding sequence.

### Brief Description of the Invention

The p53as antibodies having the utilities described may be antibodies to naturally occurring or synthetic p53as. A form of natural p53as is present in normal cells of at least some mammals. p53as is essentially identical to known normal growth controlling protein p53, at least until the final 50 and preferably until the final 30 amino acids of the carboxy terminal end of the protein. "Essentially identical" means at least 80% and preferably at least 90% sequential correspondence with p53. It should be noted that mouse p53 has an 81% identity with mouse p53as at the protein level. Mouse p53as has a highly acidic N terminus, a basic C-terminus and a central region containing uncharged amino acids

The final 50 amino acids of p53as protein proximate the carboxy terminus of the p53as protein, are at least partly different than the final 50 amino acids of p53 protein. The difference in naturally occurring p53as is believed to be at least in part due to different amino acid sequences in the two proteins proximate the carboxy termination of the protein and may also be partly due to a longer or shorter p53as amino acid chain when compared with p53. It is believed that the most common and probable final few amino acids at the carboxy termination of naturally occurring p53as contain the sequences SPNC and SPPC.

p53as has been found to function as a growth regulator in all mammals tested regardless of whether or not p53as has been found to naturally occur in the mammal.

It is to be understood that p53as may be of natural or synthetic form, provided that, at a minimum, terminal amino acids differ in whole or in part from the 50 terminal amino acids of p53 so that the modified products will act the same as active p53 protein.

In general, it can be stated that p53as is functionally the same as p53 except that a p53as lacks the negative regulatory domain for p53 sequence specific DNA binding which is found within the last 50 amino acids at the p53 C terminus. The negative regulatory domain of p53 negates p53 sequence specific binding in certain cellular environments which in turn causes p53 to lose activity. p53as lacks the negative regulatory domain and thus remains active in similar cellular environments. To obtain a p53as the terminal amino acids of p53 are preferably modified, i.e., there is at least some substitution, as opposed to simple truncation.

As further utility, evidence is presented that p53as protein has "tumor suppressor" activity in mouse and human cells, activates transcription through p53 target sequences of mouse and human cells, and forms tetramers, a DNA binding form observed for the tumor suppressor gene p53as. The invention includes a method for characterization of unknown cell products by: reacting the unknown cell products with an antibody which does not bind to a normal p53 from a mammal and which specifically binds to a p53as protein; determining the presence and concentrations of p53as in the unknown cell products as a result of the reaction of the unknown cell products with the antibody; and comparing the determined concentrations of p53as in the unknown cell products with p53as concentrations in known cell products for assistance in characterizing the unknown cell products. This Application further claims the use of antibodies to human p53as for diagnosis of human cancers, the use of antibodies to human p53as for determination of prognosis of human cancers and use of antibodies to human p53as for determining a treatment plan for individual patient cancers.

The invention also comprises plasmids and viral vectors containing a p53as cDNA sequence. A preferred viral vector is baculovirus vector. Support is provided for the utility of p53as gene expression from plasmids and vectors.

The invention includes antibodies both polyclonal and monoclonal, to p53as and to at least a portion of human p53 intron 10 sequence encoding SLRPFKALVREKGHRPSSHSC (Sequence ID # 1) which is related to p53as sequences and plasmids and viral vectors containing such sequences.

All of the above find utility in studying p53 and p53as and their relative expressions which is believed important for detection and control of malignant cells and their susceptibility to treatment agents.

The antibodies can detect the presence of p53as and related sequences and when incorporated into cells could cause cell cycle arrest and the plasmids and viral vectors, with appropriate promotors, can cause expression of the p53as and p53 intron 10 sequences which can affect cell growth and perhaps arrest certain malignancies.

### Brief Description of the Drawings

Figure 1 shows a diagram for a proposed mechanism of activation of DNA binding by p53 protein.

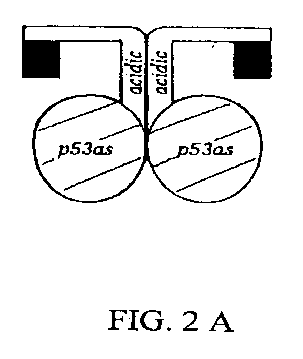

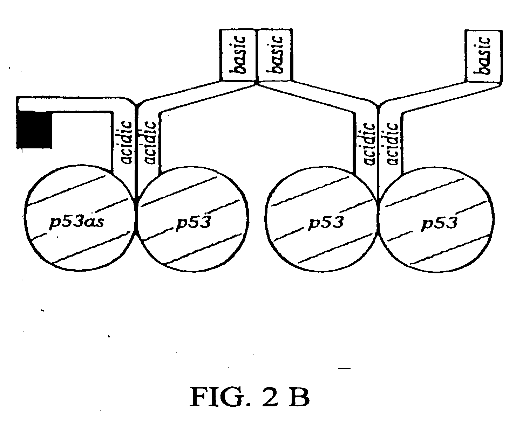

Figure 2 shows a diagram for a proposed model for p53as protein activity.

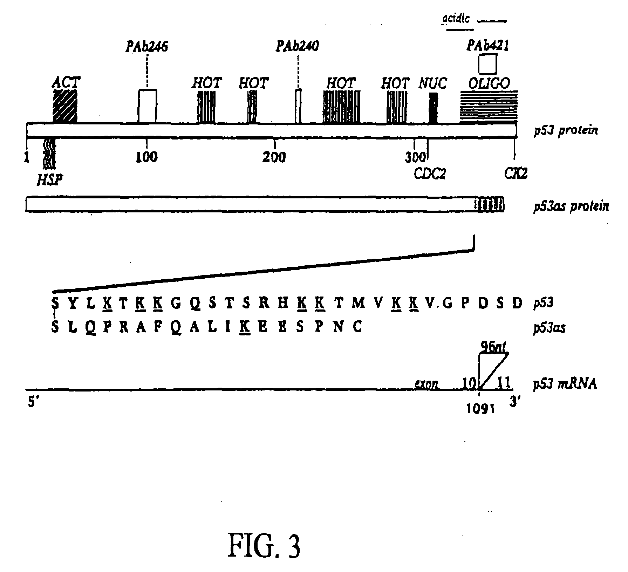

Figure 3 is a domain map of p53 protein showing changes introduced by alternative splicing (Sequence ID #'s 15 and 16). Mouse p53 has 390 amino acids. Domains (see Vogelstein et al., (1992), "p53 function and dysfunction", Cell 70, 523-526 and references therein) are ACT:transcriptional activation domain; HSP; heat shock protein binding region of mutant p53; HOT spots; highly conserved regions among p53 proteins in which most transforming mutations occur; PAb240; region binding antibody conformation-specific for certain mutants, murine amino acids 156-214; PAb246: region binding antibody to normal wt. conformation, murine amino acids 88-109; PAb421: region binding antibody to wt. and mutant conformation, amino acids 370-378; NUC:nuclear localization signal; CDC2 kinase serine phosphorylation site; CK2 casein kinase serine phosphorylation site, which is also the site of 5.8rRNA binding; OLIGO: site of p53 self-association. The expected changes in the C-terminal region of protein translated from alternatively spliced wt. (Han et al., (b) (1992), supra) or mutant p53 mRNA (Arai et al., (1986) "Immunologically distinct p53 molecules generated by alternative splicing", Mol. and Cell. Biol., 6, 3232-3239) are shown. The segment of intron 10 retained in p53as mRNA is indicated as a triangle between exons. Acidic amino acids (within a predicted alpha-helix spanning 334-356) and basic amino acids (Between position 363 and 386 - underlined in the C-terminal peptide sequence at bottom) are labeled according to Sturzbecher et al., (1992), "A C-terminal a -helix plus basic region motif is the major structural determinant of p53 tetramerization", Oncogene 7, 1513-1523.

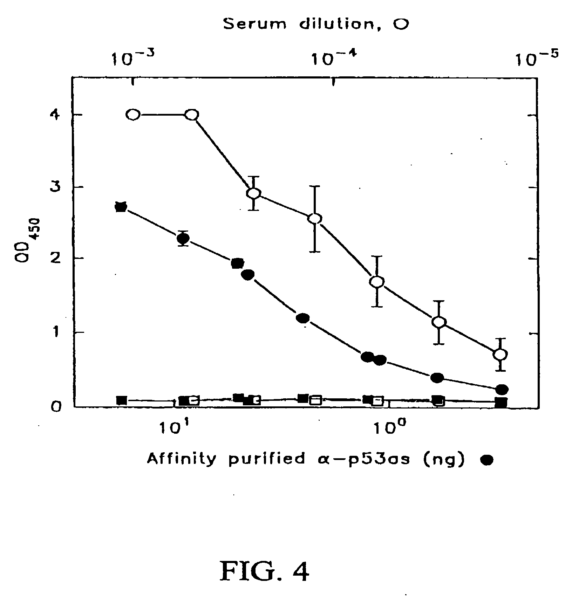

Figure 4 shows a graph of reactivities with p53as peptide of anti-p53as serum and affinity-purified antibodies detected by ELISA. New Zealand White female rabbits were immunized with a peptide equivalent to the C-terminal 17-amino acids of p53as. p53as peptide was synthesized at the RPCI Biopolymer facility and immunizations were performed at RPCI Springville Laboratories. ELISA plates were coated with 2 µg peptide and reacted with pre-immune serum or day 63 immune serum at 1/500 through 1/640,000 (1/2 dilutions) and peroxidase-conjugated, affinity-isolated goat anti-rabbit immunoglobulin. Whole immune serum (open circles) or affinity-purified (to the peptide) anti-p53as antibodies (closed circles) were used as primary antibodies with whole pre-immune serum (open squares) or ammonium sulfate precipitated IgG fraction (closed squares) were used as controls.

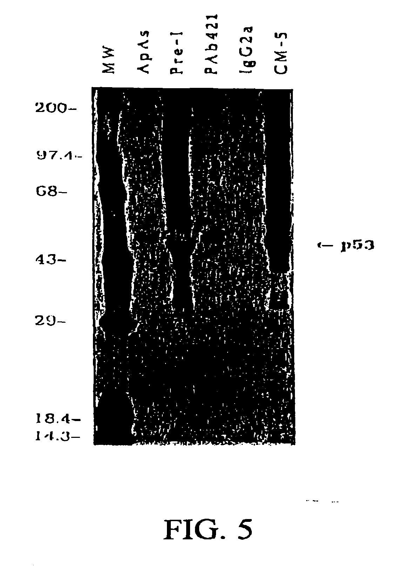

Figure 5 shows an anti-p53as immunoprecipitation of a 53kd protein. Immunoprecipitation of p53as from squamous cell carcinoma line 291.03RAT: ³⁵S methionine-labeled cells were lysed and 2 x 10⁷ cpm of lysate were reacted with the antibodies of antisera indicated: ApAs, affinity purified anti-p53as; Pre-I, pre-immune rabbit serum; PAb421 anti-p53 anti-p53 antibody to an epitope absent in p53as; IgB2a, mouse IgG idiotype control for PAb421; CM-5, rabbit polyclonal anti-p53 antibody reactive with both p53 and p53as proteins; MW, molecular weight standards (kd). After separation from the antibody complex by heating at 85°C for 5 min., proteins were resolved by electrophoresis as described in Experimental Procedures. 53kd proteins were detectable by PAb421 and affinity purified anti-p53as (ApAs) and rabbit polyclonal anti-p53 serum CM5.

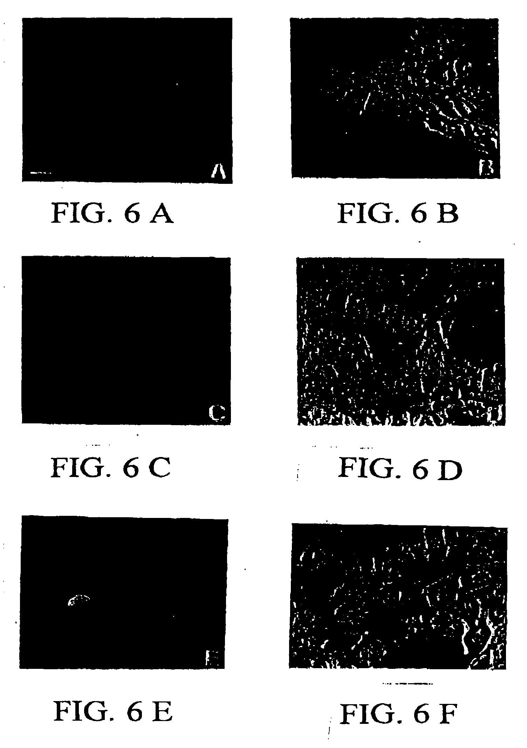

Figure 6 views A through E (labeled Figures 6A, 6B, 6C, 6D, 6E and 6F of the drawings, respectively) shows immunofluorescence fields indicating nuclear localization of p53as antigen activity. Cells were plated at 1.5x10⁴ cells/cm² on glass coverslips and grown until about 70% confluent. Nuclear reactivity was detected using affinity-purified anti-ASp53 antibody in indirect immunofluorescence assays of 100% EtOH-fixed cells (A). this reactivity was completely blocked by competition with 1:1 ratio (by weight) of the 17 amino acid peptide corresponding to the C-terminus of the p53as protein (C). No competition was evident with up to a 10:1 ratio of an unrelated 16 amino acid peptide (E). Phase contrast optics corresponding to the immunofluorescence field are shown at right (B, D, F). Findings were similar for all epidermal cell lines (transfectant clone 119 of 291.03 RAT is shown). Fluorescence in IgG2a, IgG1 or ammonium-sulfate fractionated pre-immune serum controls were negligible (data not shown). Bar equals 15 µm.

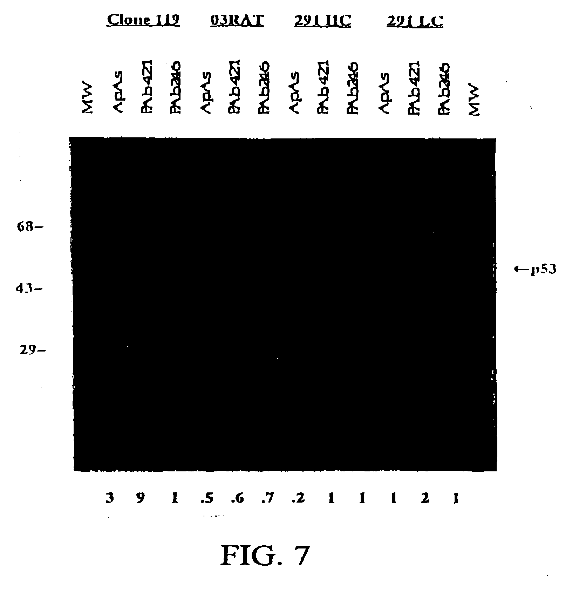

Figure 7 shows comparative immunoprecipitation of p53 from proliferating (LC) or differentiating (HC) nontransformed parental 291 cells and from 291.03RAT (03RAT) carcinoma cells or its derivative clone 119 transfected with a mutant p53 (valine-135). Cell lysates are incubated with 2µg PAb421, 4µ PAb246 or 1.4µ anti-p53as (ApAs) in NET/GEL. Immunocomplexes were incubated with 5mg protein A for 2h at 4°C, centrifuged and immunoprecipitated protein was eluted from pellets with heating at 85°C for 15 min. After centrifugation proteins in the supernatants were separated by electrophoresis on a 10% polyacrylamide denaturing gel with molecular weight standards (MW, kd). Immunoprecipitable p53as protein in clone 119 could have resulted from transfected mutant transcripts or from endogenous wt. p53as RNA. To assist in comparisons of a particular antibody reactivity among the cell lines, the densities of the p53 signal in each lane are provided (numbers at bottom of each lane) relative to ApAs reactivity of 291LC as 1.

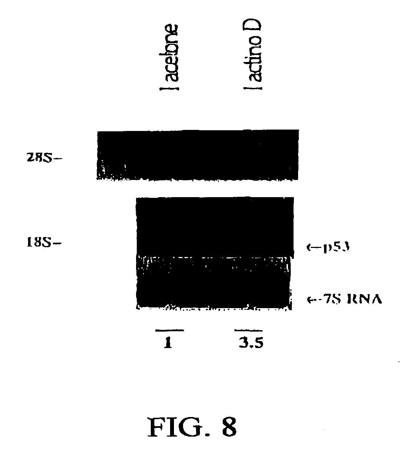

Figure 8 shows a northern blot of p53 RNA in 291.05 RAT carcinoma cells following treatment with actinomycin D. Cells were harvested after exposure to 0.5nM actinomycin D or 0.2% acetone for 48h and RNA was extracted as detailed in Experimental Procedures.

Fold-increase in signals detectable by densitometry, adjusted for loading by comparison with 7S RNA, are indicated.

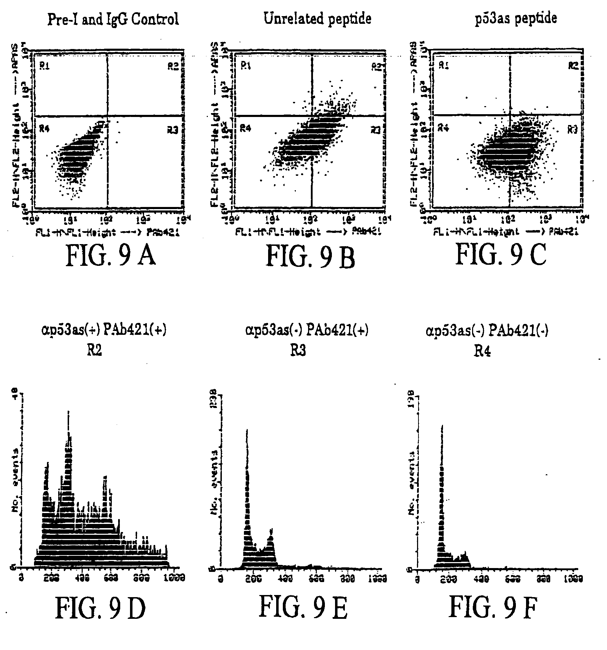

Figures 9A through 9F show expression of p53 (PAb421) and p53as antigen activities in 291.05RAT carcinoma cells. Cells were treated with 0.5nM actinomycin D for 2 days and harvested by trypsinization. Cells were permeabilized and stained in suspension with anti-p53as and PAb421 antibodies (in the same tube) in all cases shown except for the primary antibody control (upper left). In the 3 dot plots at top, Figures 9A through 9C, the FL1 fluorescence intensity on the x-axis was FITC (green), used to visualize PAb421 reactivity, and the FL2 fluorescence intensity on the y-axis was phycoerythrin (red) used to visualize anti-p53as reactivity. Prior to incubation with cells, the anti-p53as antibody was exposed to either p53as peptide, which competitively removed the specific anti-p53as reactivity, or to an unrelated peptide, which controlled for nonspecific binding to peptide, leaving only specific reactivity to p53as protein. Events collected by flow cytometry were single cells only as described in Experimental Procedures. Coordinates were set on total cell data based on IgG2a and pre-immune controls to delineate four regions: negative for both antibodies (R4), positive for anti-p53as only (R1), positive for PAb421 and anti-p53as (R2) and positive for PAb421 only (R3). After collecting a file of 10,000 total events per tube (as shown in the 3 dot plots at top), additional gates were set to exclude negative cells (R4) and to maximize the collection of cells positive for PAb421 (histogram R3 shown), or to exclude negative cells (R4) and cells positive only for PAb421 (R3) in order to maximize the collection of cells positive for anti-p53as (R2). The cell cycle distribution of events from each region (R2 through R4, Figures 9D through 9F) is shown in the 3 histograms at bottom. The numbers of cells in each region expressed as a percentage of the total cells were: unrelated peptide, R1, 0.02, R2, 1.7, R3, 17 and R4, 79 (numbers may not add to 100 due to rounding error and to a negligible number of events outside the windows included in the analysis); p53as peptide, R1, 0.02, R2, 0.05, R3, 19, R4, 79. Percentages of cells in each phase of the cell cycle and total events (n) for each region in the histograms shown were: R2, G0/G1, 19, S, 13, G2/M, 25 and .G2/M, 43, n=2224; R3, G0/G1, 38, S, 16, G2/M, 33 and .G2/M, 12, n=6879; R4, G0/G1, 60, S, 18, G2/M, 21 and .G2/M, 1, n=7925.

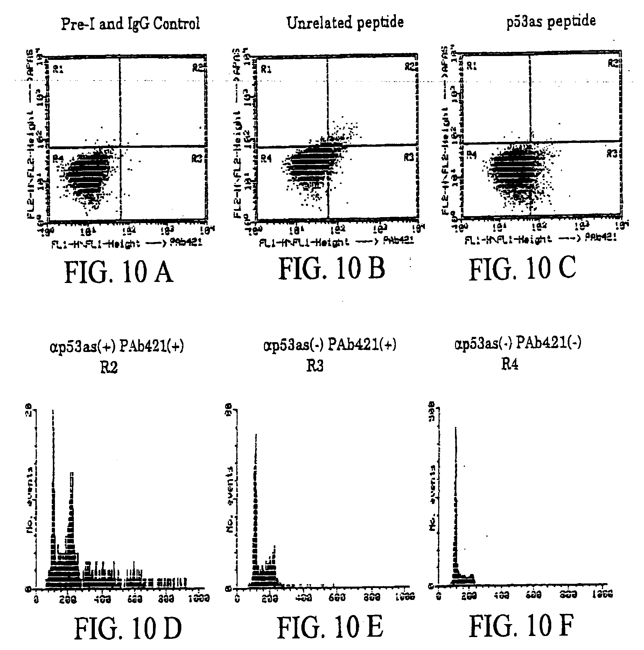

Figures 10A through 10F show expression of p53 (PAb421) and p53as antigen activities in 291.05RAT carcinoma cells. Cells were cultured under low Ca²⁺ conditions (LC) which favored cell growth. Treatment and analysis was the same as for carcinoma cells presented in Figures 9A through 9F. The dot plots are shown in Figures 10A through 10C and the histograms are shown in Figures 10D through 10F. The numbers of cells in each region expressed as a percentage of the total cells were: unrelated peptide. R1, 1.2, R1, 2, R3, 4 and R4, 92; p53as peptide, R1, 0.1, R2, 0.1, R3, 5, R4, 94. Percentages of control cells in each phase of the cell cycle and total events (n) for each region in the histograms shown were: R2, G0/G1, 29, S, 11, G2/M, 35 and .G2/M, 25, n=1074; R3, G0/G1, 61, S, 15, G2/M, 22 and .G2/M, 2, n=1974; R4, G0/G1, 78, S, 11, G2/M, 11 and .G2/M, 0.04, n=9194.

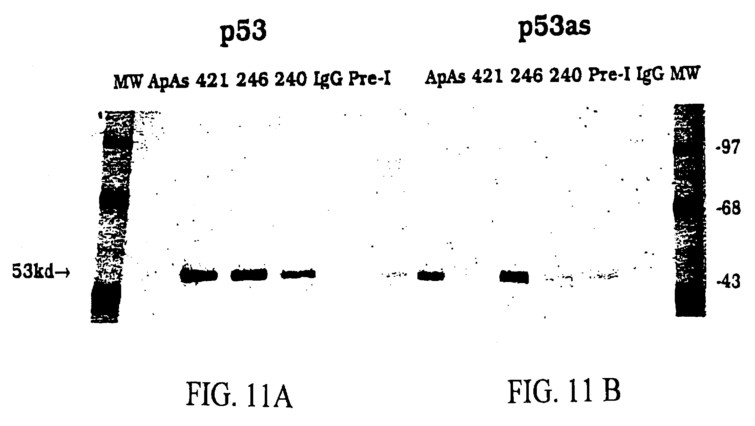

Figures 11A and 11B show immunoprecipitation of proteins translated *in vitro*: Figure 11A shows reactions for p53 and Figure 11B shows reactions for p53as, respectively. PAb421 is specific for the major p53 form only, and ApAs is specific for p53as protein only. 10µg of plasmid pBSp53as or pBSp53 was linearized by BamHI and transcribed with 20 Units T3 RNA polymerase. The *in vitro* translation was performed according to a standard protocol (Promega) by incubating 3µg RNA with 35µl of rabbit reticulocyte lysate in the presence of 4µl (40µCi) ³⁵S-methionine for labeling of proteins. For co-translation of p53as and p53, an equal amount of each RNA was incubated with rabbit reticulocyte lysate (Promega). Immunoprecipitation was performed by incubating 5µl ³⁵S-labeled protein with 1µg PAb421, ApAs or PAb246 at 4°C overnight. Protein A-Sepharose 4B was then added with gentle mixing at 4°C for 2 hr. After centrifugation, the pellets were washed three times with Net-gel buffer. The pellets were suspended in 2x sample buffer containing 100mM DTT and separated by electrophoresis in a 7.5% SDS-polyacrylamide minigel. The gel was enhanced, dried and exposed overnight to Kodak X film. Two lysate reactions are shown, p53 or p53as RNA - see labels at top. Antibodies used to immunoprecipitate protein from each lysate reaction are indicated.

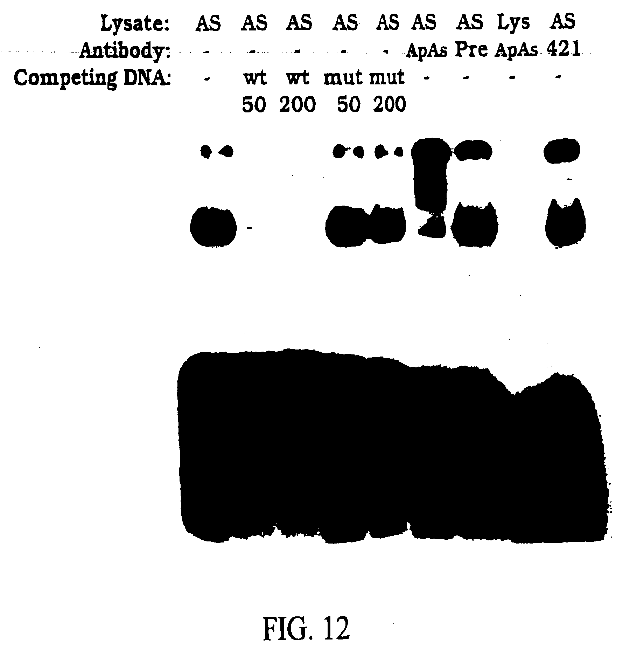

Figure 12 through Fig. 16 represent electrophoretic mobility shift assays (EMSA) of p53 and p53as proteins translated *in vitro* using the DNA probe sequence presented in Table 2.

Figure 12. p53as protein binds specifically and efficiently to DNA. Binding of p53as protein translated *in vitro* (5 µl of 250 µl reaction) to ³²P-labeled oligonucleotide (ng) in the presence of increasing amount of unlabeled wt p53 binding sequence (wt) or of mutated (mut) sequence (see [1] in Materials and Methods) demonstrates the specificity of p53as protein for the p53 binding motif. 200ng ApAs antibody to p53as can super-shift the binding complex. 200ng of pre-immune rabbit serum (Pre) and PAb421 (421) were used as controls.

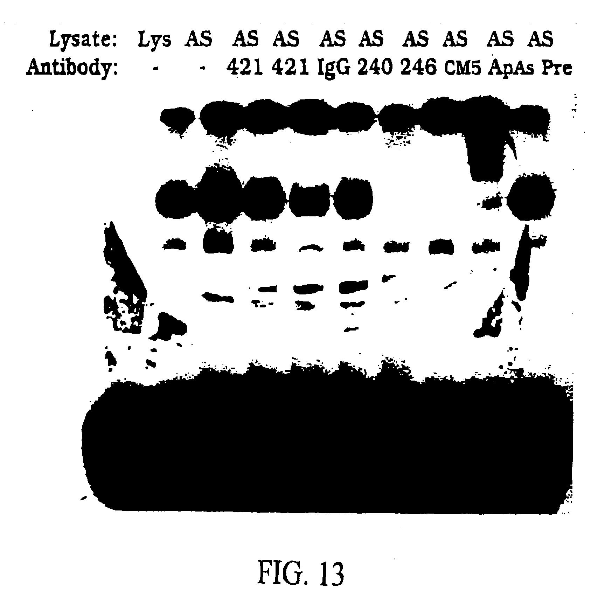

Figure 13. p53as protein complex with DNA is shifted by anti-p53as (ApAs) and abrogated by anti-p53 antibodies PAb246 (246) and CM5 but not mutant conformation-specific PAb240 (240). p53as protein was translated *in vitro* in reticulocyte lysates and assayed by EMSA for binding to ³²P-labeled probe in the absence of antibodies (-) or in the presence of PAb421, 200ng PAb246 and rabbit polyclonal anti-p53 antibody CM5, or 200 ng ApAs. IgG2a (IgG), pre-immune serum (Pre), and non-programmed reticulocyte lysate (Lys) were used as controls.

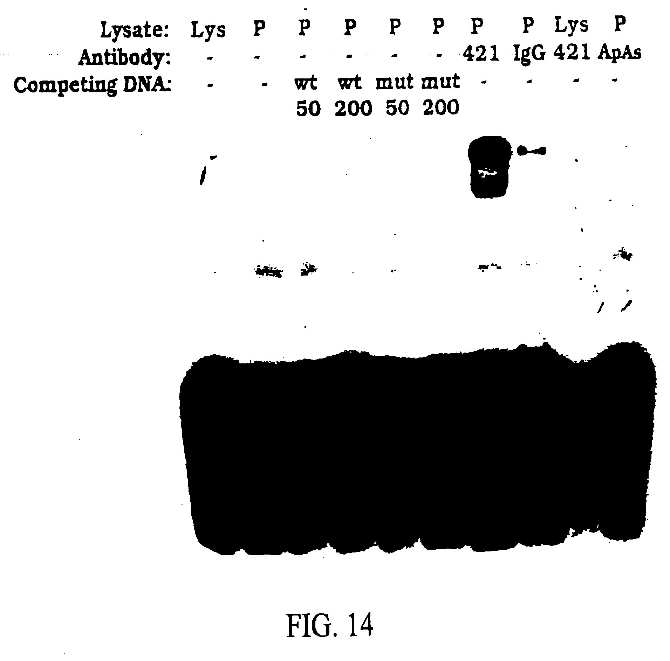

Figure 14. p53 protein requires activation to bind to DNA. There is no apparent binding to the ³²P-labeled probe by p53 (lanes 2-6, 8-10) as compared to the non-programmed rabbit reticulocyte lysate (lane 1). A binding complex can be detected when 200ng of PAb421 was included in the reaction (lane 7).

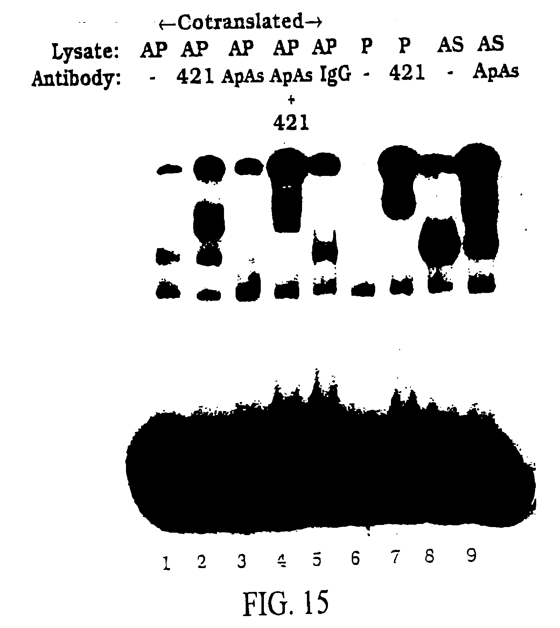

Figure 15. Interaction of p53as with p53 and its effects on DNA binding activity of p53as. Equal amount of *in vitro* transcripted p53 and p53as RNA's were co-translated (AP) or translated individually and p53 (P) and p53as (AS) proteins were assayed for their DNA binding activities by EMSA. The co-translated proteins have lower DNA binding activities (lane 1) than p53as alone (lane 8). Two binding complexes can be detected when 200ng PAb421 (lane 2) or 200ng PAb421 plus ApAs (lane 4) were included in the reactions containing co-translated proteins, while only one binding complex appears as PAb421 activates p53 DNA binding activity (lane 7). 200ng ApAs did not cause any apparent supershift for co-translated proteins (lane 3). Two higher molecular weight binding complexes can be seen when ApAs was added to the reaction containing only p53as protein (lane 9).

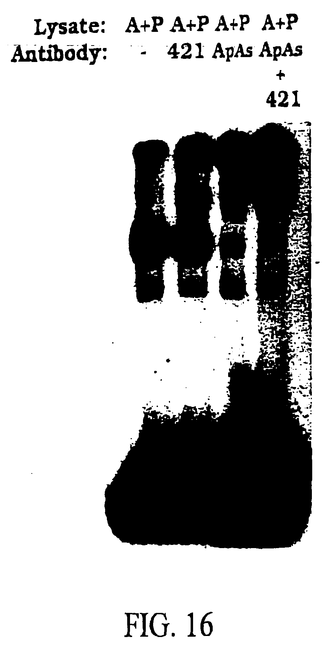

Figure 16. Lack of interaction of p53as and p53 proteins mixed posttranslationally. Equal amounts of p53 and p53as translated *in vitro* were mixed and assayed for binding to ³²P-labeled oligonucleotide by non-denaturing polyacrylamide gel electrophoresis (lane 1). 200ng each of the indicated antibodies were added to the binding reaction.

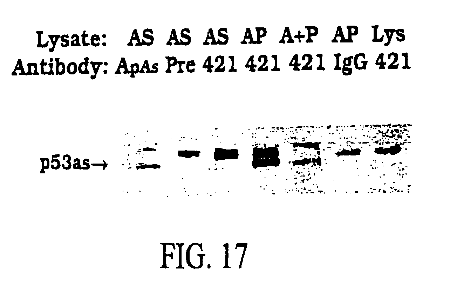

Figure 17. Heteroligomers of p53 with p53as identified by immunoprecipitation followed by immunoblotting. *In vitro* translated proteins were p53as (AS), p53 (P), cotranslated p53 and p53as (AP) or a mixture of p53as plus p53 translated individually (A+P) and non-programmed reticulocyte lysate control (Lys). The translated proteins (15µl each lysate, 30µl for cotranslation) were immunoprecipitated with anti-p53 antibody PAb421 (421) rabbit antibody to p53as (ApAs), pre-immune (Pre) or IgG2a (IgG) controls. The immune complexes were fractionated on a 10% SDS-polyacrylamide minigel, transferred to a nitrocellulose membrane and incubated with horseradish peroxidase-conjugated ApAs for 1 hour. The p53as was visualized through chemiluminescence (ECL, Amersham, Arlington Heights, IL.).

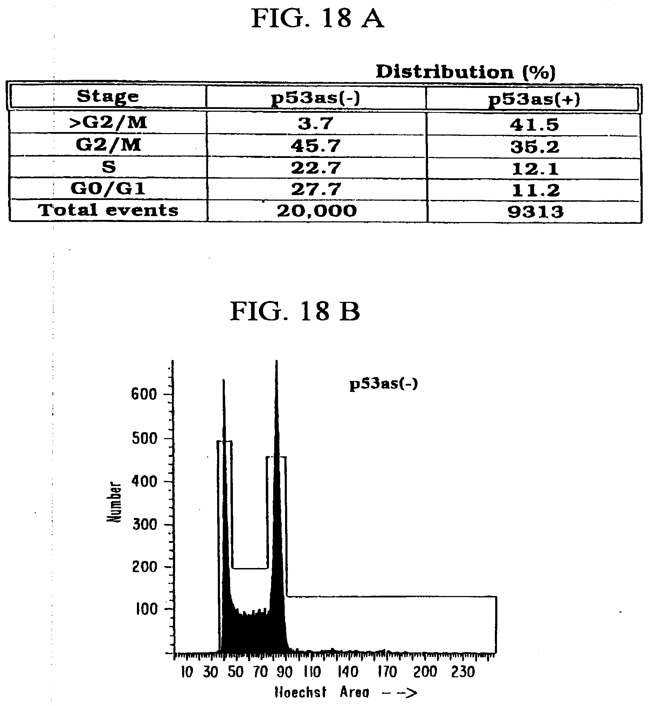

Figure 18A is a table showing percent distribution of p53as reactivity at various stages of insect cell cycles after infection with baculovirus vector containing the full length p53as cDNA. Insect cells arrest in G2 phase of the cell cycle after infection with the baculovirus vector.

Figure 18B is a graph showing p53as(-) distribution for insect cells.

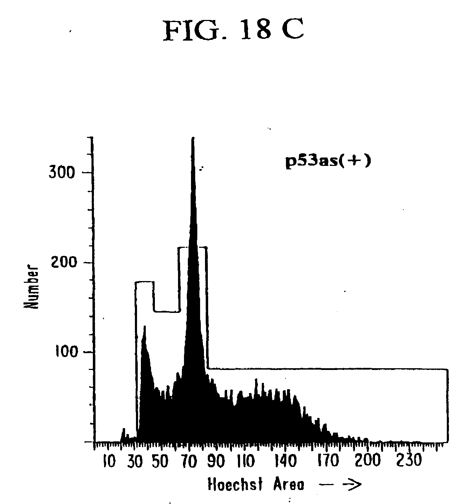

Figure 18C is a graph showing p53as(+) distribution for insect cells.

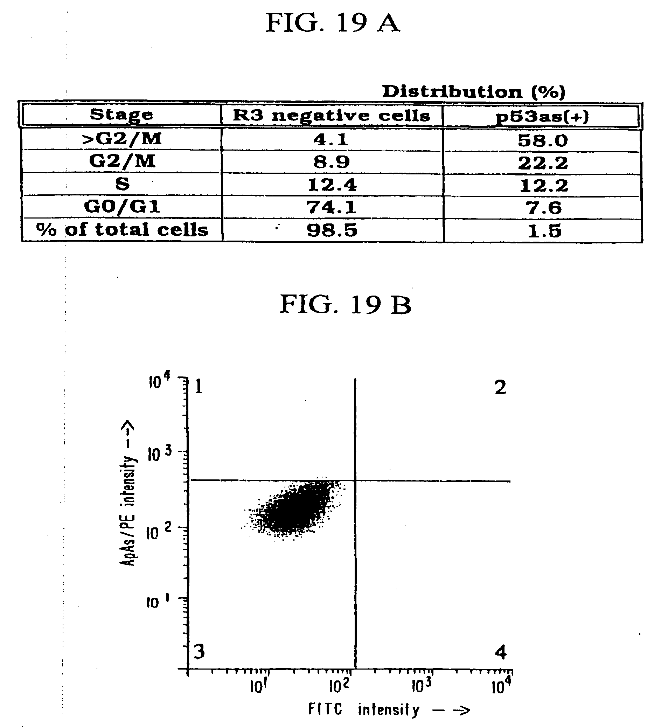

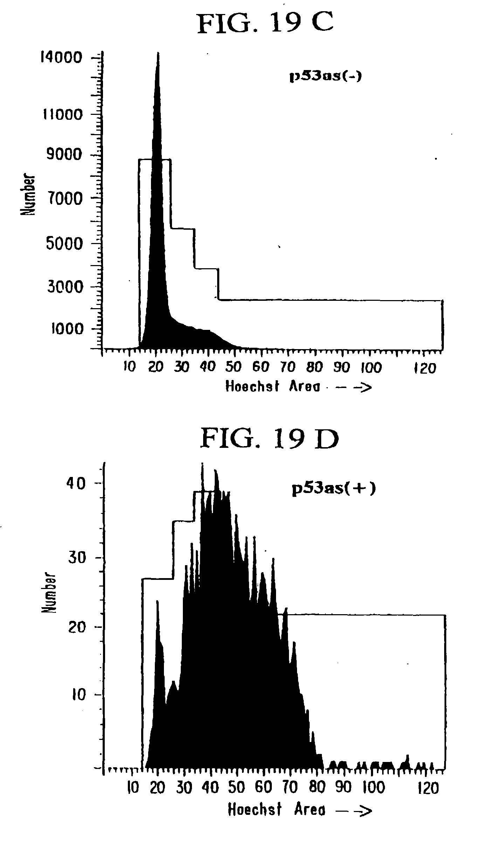

Figure 19A is a table showing percent distribution of cells in various stages after being transfected with plasmid containing p53as cDNA and showing that transfected cells were preferentially distributed in the G2 phase of the cell cycle. The cells used are mouse squamous cell carcinoma cells which arrest in the G2 phase of the cell cycle after transfection with a plasmid construct containing p53as cDNA.

Figure 19B shows distribution of ApAs/PE intensity vs. FITc intensity for mouse squamous cells.

Figure 19C is a graph showing p53as(-) for transfected mouse squamous cells.

Figure 19D is a graph showing p53as(+) for transfected mouse squamous cells.

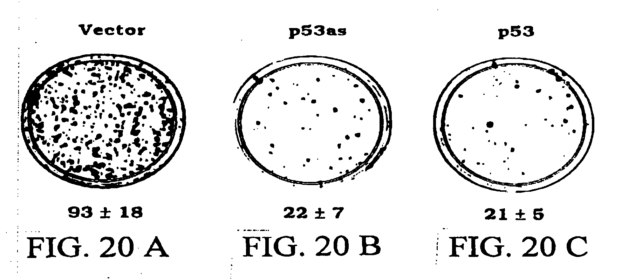

Figures 20A, 20B and 20C show colony formation of Saos-2 cells transfected with CMV plasmids. Figure 20A shows a control without a cDNA insect. Figure 20B shows a p53as cDNA insect and Figure 20C shows a p53 cDNA insect. Plasmid containing p53as containing a CMV promoter (which drives expression of p53as in the target mammalian cell) is introduced into human osteosarcoma cells which lack p53 which stops tumor cell growth and reduces the number and area of colonies present in the culture dishes. This assay is commonly used to show tumor suppressor activity of various genes and p53 used as a control shows similar activity, thus wild type p53as acts as a tumor suppressor. In contrast, a plasmid containing an alternatively spliced mutant p53 sequence had transforming activity (Eliyahu et al., Oncogene 3,313-321, 1988.). Tumor suppressor activity was found in human cells as well as mouse cells. Human osteosarcoma cell line Saos-2 was transfected using lipofectin, BRL0 with 5 ng of the pCMV plasmids containing the p53 or p53as cDNA indicated and a neomycin resistance gene. Vector without the cDNA insert was used as a control. Forty-eight hours after transfection, cells were trypsinized, passaged at a 1:4 ratio and cultured in media containing 500 µg/ml G418 for 3 weeks. Colonies were fixed in methanol and stained with Giemsa and measured using an image analysis system (Spectra Services).

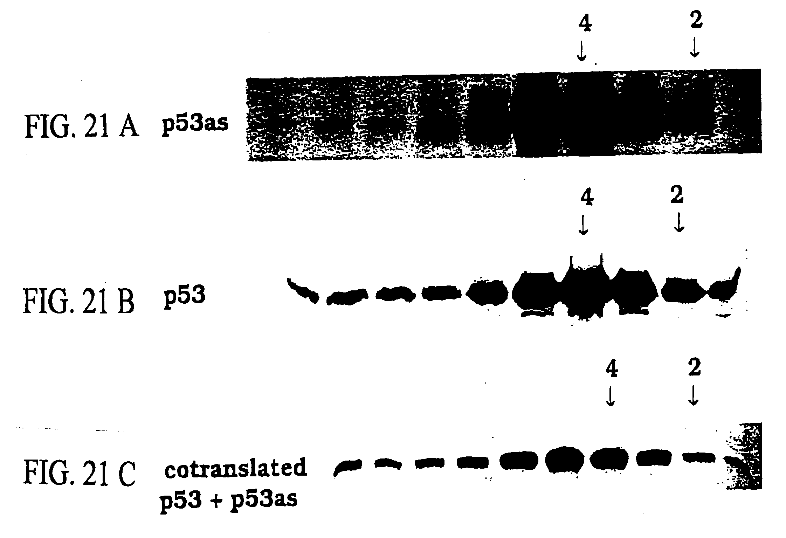

Figures 21A, 21B and 21C show gel filtration profiles of proteins translated *in vitro*. The *in vitro* translations were carried out as described in parent application 08/195,952. p53as (Figure 11A), p53 (Figure 11B) and cotranslated p53 and p53as (Figure 11C) were fractionated by gel filtration (FPLC) on a Superose 6 column (Pharmacia) with column buffer (0.4M NaCl, 0.5% NP-20, 20 mM Tris-HCl, pH 7.2) at a flow rate of 0.4ml/min. 0.4 ml eluant was collected in each fraction. The ³⁵S-labeled proteins in each fraction were visualized by SDS-PAGE and autoradiography. Black arrows indicate the predicted positions of tetramers (4) and dimers (2) based on the positions of molecular weight standards, amylase (200 kd) and phosphorylase B (97 kd).

### Detailed Description of the Invention

"p53as protein for a mammal", as used herein, means a natural or synthetic protein having at least 80 and preferably at least 90% identity with p53 protein from that mammal and which has the same sequence specific binding on the cellular level as the active p53 protein for that mammal, except that all of the sequence specific binding remains active in cellular environments in which p53 sequence specific binding is deactivated. p53as protein does not contain the p53 negative regulatory domain. The p53as of the invention is usually longer or shorter than p53 protein and differs from p53 protein within the final 50 carboxy terminal amino acids, and contains a specific antigen site within its final 50 carboxy terminal amino acids which gives rise to an antibody unique for the p53as. The antibody to the specific site, binds to the p53as without binding to the p53. p53as protein may also be referred to as alternatively spliced p53.

"p53as antibody" is the antibody which specifically binds to p53as protein without binding to p53 protein.

"p53as gene" means a gene encoding p53RNA.

"p53RNA" is RNA which encodes p53 protein.

"p53asRNA" is RNA which encodes p53as protein.

A novel form of a natural wild type (normal) p53 protein is present in nontransformed mouse cell strains and mouse squamous cell carcinomas. Designated p53as, (alternatively spliced p53) it arises from a normal variation in processing of the p53 messenger RNA (mRNA).

It has been demonstrated that a wild type alternatively spliced p53 (p53as) RNA exists in mouse cultured cells and normal mouse tissue at approximately 25 to 33% of the major p53 RNA form. It has been found that the alternative RNA transcript is 96nt longer than the major transcript due alternative slicing of intron 10 sequences. It has now been determined that the natural p53as protein exists in nontransformed and malignant mouse epidermal cells and is localized to the nucleus along with the major p53 protein. The protein generated from the p53as transcript is 9 amino acids shorter than the major p53 protein and has 17 different amino acids at the carboxyl terminus. In addition, p53as protein is preferentially expressed during the G2 phase of the cell cycle and in cells with greater than G2 DNA content, compared to the major p53 protein which is preferentially expressed in G1. The p53as immunoreactivity is elevated and shifted to the G1 phase of the cell cycle following actinomycin D treatment of nontransformed but not malignant cells. In view of the dimerization and tetramerization of p53 protein which may be necessary for its DNA binding and transcriptional activation activities, the presence of p53as protein in cells has important implications for understanding the physiological function(s) of the p53 gene.

It is believed that the p53as protein, natural and synthetic, similar to p53, may be used in studying cell growth and maturation, detecting normal versus abnormal cell growth and may be used to normalize cell growth of abnormally growing cells.

Hereafter the p53 protein recognized to date will be referred to as the major form of p53 or simply p53; this does not rule out the existence of a cell type in which p53as may be present in relatively higher amounts than p53. DNA is transcribed to RNA which is then processed by removal of segments called introns to give the mature messenger RNA which encodes protein. In alternative splicing, a segment of an intron is retained in the coding sequence (called an alternatively spliced messenger RNA) and makes a protein which is partly different from the major form of the protein. The p53as sequence found in mouse normal and tumor cells has 17 different amino acids at the carboxyl terminus of the p53 protein (out of a total of 390 amino acids in the major p53 form). This unique sequence was used to generate antibody specific to mouse p53as. The antibody does not cross react with the major p53 protein.

The natural mouse p53as protein, by virtue of the replacement of 17 amino acids and loss of 9 amino acids within the carboxyl terminus, already harbors changes in this "DNA activation" region. p53as protein in complex with itself (dimer formation is possible due to retention of acidic residues known to be important for dimerization of p53 protein) or in complex with the major p53 protein will bind to DNA and modulate transcription of specific target genes with altered, perhaps greater, efficiency than homodimers or homotetramers of the major p53 protein only (see Figure 2).

Polyclonal and monoclonal antibodies available prior to the present invention do not specifically and selectively recognize p53as; although antibodies exist which recognize p53. These include PAb246 which recognizes mouse p53 in its normal folding state, PAb240 which recognizes certain mutant p53 proteins, PAb421 which recognizes the carboxyl terminal amino acids replaced or lost in p53as. Similarly, human p53 is recognized by monoclonal and polyclonal antibodies. No report of a human p53 generated by alternative splicing at the carboxyl terminus has been reported and no specific antibody to human p53as is available.

It has now been found that a synthetic form of p53as may readily be made by deleting or altering the negative regulatory domain within the final 50 carboxy terminal amino acids of p53. The synthetic p53as acts as an active form of p53 even in cellular environments where p53 is normally deactivated through its negative regulatory sequence.

In accordance with the present invention the p53as for a mammal is provided with a unique antigen site to which an antibody may be formed which recognizes the p53as but does not recognize p53 from the mammal.

The development of antibody to p53as protein in accordance with the present invention, permits studies of whether p53 protein and p53as protein associate in the cell, and will permit *in vitro* studies of the efficiency of DNA binding and transcriptional regulation of complexes between p53 and p53as proteins. Such antibodies may also permit detection of cells having normal growth from cells having abnormal accelerated growth.

### Mouse p53as peptide

Alternative splicing of mouse p53 RNA results in insertion of 96nt from intron 10 of the p53 gene. These 96nt encode (in frame) 17 amino acids which are distinct from those in the major p53 RNA form, beginning at residue 365 and extending to residue 381, followed by a stop codon which results in truncation by 9 amino acids. This 17 amino acid peptide of alternatively spliced mouse p53, called mouse p53as peptide is: LQPRAFQALIKEESPNC (Sequence ID # 2). It was produced by standard synthesis, tested for authenticity and is stored in the laboratory. Details and procedures are as follows:

During the sequencing of p53 cDNA from the tumor and normal cells of the mouse cloned keratinocyte model the inventor herein detected an alternatively spliced p53 mRNA in which 96nt of the 3' end of intron 10 are inserted between nt 1091 and nt 1092 of the mouse p53 gene (1 being adenine of the first ATG codon; (Han et al. (b) (1992), supra). p53 mRNA was first cloned as a mutant p53 cDNA (M-8) from a chemically transformed fibroblast cell line by Wolf et al., (1985) "Isolation of a lull-length mouse cDNA clone coding for an immunologically distinct p53 molecule", Mol. and Cell. Biol. 51, 127-132: Arai et al., supra, reported the sequence of this p53 cDNA variant, confirming its origin by alternative splicing. It appeared to be specific to this tumor cell lineage because it was undetectable in a nontransformed helper T-cell cDNA library. However, it has been demonstrated that wild type alternatively spliced 53 RNA is expressed in normal cells and tissues at about 25 to 33% of the major p53 RNA form (Han et al., (b) (1992) supra). In addition, it is present at approximately the same ratio in the two independently-derived epidermal carcinoma lines which overexpress p53 RNA (noted above), and thus appeared to be coordinately elevated with the major form of p53.

The translation of alternatively spliced p53 results in the substitution of 17 amino acids and in truncation of the regularly spliced form of p53 by 9 amino acids (Fig. 3). The protein translated from the alternatively spliced p53 RNA lacks the serine-389 casein kinase II and RNA binding site, the epitope for PAb421 p53 antibody binding and the basic oligomerization domain, with the potential for profound effects on p53 oligomerization, DNA binding and transcriptional activation.

It has been found, by the inventor herein, that the alternatively spliced wt. p53 protein (designated herein as p53as) exists in normal and tumor cells of a mouse epidermal cell transformation model and is differentially expressed during the cell cycle relative to the major p53 form. The presence of this physiological form of p53 protein in cells has important implications for normal p53 function and p53 inactivation in malignancy.

In order to determine whether the p53as protein was made in cells, a polyclonal antibody to the 17 amino acid sequence unique to the mouse p53 as was generated in rabbits. Rabbit serum collected at intervals after immunization was tested for reactivity to p53as peptide coated on ELISA plate wells (Fig. 4). High titer serum (shown) was affinity-purified against the 17 amino acid peptide. The reactivity (per µg antigen) of 10ng affinity-purified antibody was approximately equivalent to a 1/40,000 dilution of whole anti-p53as antiserum. Anti-p53as reactivity in the ELISA and indirect immunofluorescence assays was blocked competitively by pre-incubation of antibody with the p53as peptide.

### Immunoprecipitation

In order to determine its reactivity with cellular proteins, affinity-purified antiserum to the natural mouse p53as was reacted with mouse epidermal cell lysates (Fig. 5). A 53 kd protein was immunoprecipitated by anti-p53as. This protein migrated slightly faster on 10% polyacrylamide gels than p53 protein immunoprecipitated by PAb421 (which binds to a carboxyl terminal epitope absent in p53as). Rabbit polyclonal anti-p53 antibody CM5 recognized a broader band spanning the region containing more discrete PAb421- and anti-p53as-reactive forms.

### Indirect immunofluorescence

The location and incidence of expression of p53as in cell populations grown on coverslips was determined by indirect immunofluorescence. As shown in Figs. 6A - 6F, nuclear staining was observed with affinity-purified anti-p53as antibody. This activity was completely blocked by competitive binding with p53as peptide (Fig. 6C). Anti-p53as antibody reactivity in 291 nontransformed cells and carcinoma cells was always nuclear under the conditions of these assays (data for clone 119 is shown), and in this respect, was like PAb246 antibody reactivity which recognizes the tumor suppressor conformation of p53. This was true even in clones of 291.03RAT transfected with the pmMTval-135 temperature sensitive mutant of p53 in which PAb421 reactivity was cytoplasmic as well as nuclear. These results suggest that, like the major p53 form, wt. p53as protein exerts its effects primarily in the nucleus.

### p53 expression in nontransformed cells and tumor cells

Squamous cell carcinoma 291.03RAT expresses 3-fold more p53 mRNA and up to 10-fold less p53 protein (PAb421 and PAb246 antibody reactivity) than the progenitor 291 cells (Han et al. (a) (1992), "Altered expression of wild-type p53 tumor suppressor gene during murine epithelial cell transformation", Cancer Research 52, 749-753). Comparison of the expression of p53as protein in these cell lines was done by immunoprecipitation. As shown in Fig. 7, reactivity with anti-p53as antibody was detected in nontransformed 291 cells and carcinoma cells. The p53as-precipitable protein in these cell lines migrates slightly faster than the PAb421 and PAb246-precipitable proteins, as expected from the truncation of p53as by 9 carboxy-terminus amino acids (expected to result in an approximately 1 kd difference in molecular weight). As expected from previous studies (Han et al. (a) supra) immunoreactivity to all three anti-p53 antibodies was lower in 291.03RAT carcinoma cells than normal cells. The ratio of immunoprecipitable protein in populations of proliferating cells vs. differentiating 291 cells was higher for anti-p53as (5/1) and for PAb421 (ratio of 2/1) than PAb246 reactivity (1/1). Elevated PAb421 reactivity in proliferating populations also was noted by Milner (1984), "Different forms of p53 detected by monoclonal antibodies in non-dividing and dividing lymphocytes", Nature 20, 143-145, in studies of mouse lymphocytes. The present results suggested that p53as protein might be differentially expressed relative to PAb421 and PAb246 protein, dependent upon cellular proliferative or differentiative states.

### Response to Actinomycin D

p53 protein has been postulated to participate in a cell cycle checkpoint regulating entry into S phase after exposure of cells to DNA damaging agents such as actinomycin D. Cells expressing wt. p53 (PAb421 reactivity) arrest in the G1 stage of the cell cycle following DNA damage and p53 immunoreactivity is coordinately increased. Prior to studies of the cell cycle distribution of p53as-positive epidermal cells, experiments were performed to determine whether p53as protein also may respond to DNA damage, whether it was possible thereby to maximize the percentage of p53as-positive cells in the cell population, and to compare the response of nontransformed and malignant epidermal cells. Moderately-differentiated squamous cell carcinoma line 291.05RAT was used for these studies because it expressed higher levels of immunoprecipitable p53 protein than 291.03RAT, but like 291.03RAT is derived from epidermal clone 291 and has the wt. p53 gene (Han et al. (a) supra). Treatment with actinomycin D induced p53 and p53as protein expression in two separate experiments, based on the percentage of cells positive for reactivity with p53 antibodies by indirect immunofluorescence (Table 1). The increase in positive cells was less for p53as than for PAb421 and PAb246 reactivities and required a higher concentration of actinomycin D, but this may reflect the lower abundance of p53as protein. As in untreated 291 epidermal cells and in the 291.03RAT tumor cells expressing wt. p53, the p53as antibody reactivity in actinomycin D-treated cells was nuclear. The p53as-positive nuclei were also positive for PAb421 or PAb246, whereas most PAb421(+) or PAb246(+) cells were negative for p53as antibody reactivity. The abundance of p53 RNA in actinomycin D-treated 291.05RAT cells was increased over 3-fold according to northern blot analysis (Fig. 8). In an independent cell preparation, reverse transcriptase-polymerase chain reaction (RT-PCR) was performed using primers which amplify a segment from nt 1042 to 1539 including the C-terminus coding sequences of p53 or p53as as described previously (Han et al. (b) supra). Both p53 and p53as transcripts were increased coordinately in samples from actinomycin-D treated cells compared to controls (data not shown), suggesting that the response of epidermal cells to actinomycin D involve increases in RNA abundance. The increase in abundance of p53 antibody reactivity following actinomycin D treatment was similar to the response of ML-1 and normal myeloid progenitor cells to γ-ray reported by Kastan et al. (1991), "Participation of p53 protein in the cellular response to DNA damage", Cancer Research, 51, 6304-6311. However, the abundance of p53 RNA was not increased in response to γ-ray and the authors suggested that the observed changes in p53 immunoreactivity resulted from a posttranscriptional mechanism. The current findings are consistent with a functional role for p53as protein along with p53 protein in cellular response to actinomycin D.

### Flow cytometry

The elevation of wt. p53 coordinated with G1 arrest of cells in response to various DNA damaging agents suggested a role as a G1/S cell cycle checkpoint permitting time for repair of DNA damage or induction of programmed cell death in severely damaged cells (Lane, (1992), p53, "Guardian of the genome", Nature 358, 15-16). Flow cytometry was performed in order to determine whether p53as antigen activity was differentially expressed during the cell cycle. Cells which had been exposed to actinomycin D or solvent were stained with antibodies to p53as and PAb421 or PAb246, taking advantage of the different species of origin of the polyclonal and monoclonal antibodies to permit immunodetection of p53as and p53 antigens in the same cell. Phycoerythrin (red) conjugated to anti-rabbit immunoglobulin was used to recognize p53as and FITC (green) conjugated to anti-mouse immunoglobulin was used to recognize PAb421 and PAb246. The specificity of anti-p53as is demonstrated in Figs. 9A - 9F. Coordinates were set based on fluorescence intensity to divide detected events (single cells) into those positive for p53as alone (region 1, R1), positive for p53as and PAb421 or PAb246 (R2), negative for p53as and positive for PAb421 or PAb246 (R3) and negative for both anti-p53as and PAb421 or PAb246 (R4). The carcinoma cells were rarely positive for anti-p53as alone (Fig. 9A). Cells positive for anti-p53as also were positive for PAb421 or PAb246. Competition with p53as peptide, but not an unrelated peptide, completely blocked events detectable in the R2 region (shown for 291.05RAT in Figs. 9A - 9F and for 291 cells in Figs. 10A - 10F), without reducing the percentage of R3 events, verifying the specificity of the anti-p53as antibody. Events from each region R1 through R4 were collected in quantity (see Figs. 9A - 9F) for analysis of cell cycle distribution, represented in the histograms (Figs. 9A - 9F and 10A - 10F). The distribution of actinomycin D-treated 291.05RAT cells (shown) and control cells were essentially the same. PAb421(+)/p53as(-) 291.05RAT carcinoma cells were distributed primarily in the G0/G1 phase of the cell cycle, while p53as (+)/PAb421(+) cells were preferentially in the G2/M phase of the cycle (Fig. 7). Particularly striking is the distribution of p53as(+) cells in a "tail" indicating DNA content in excess of G2/M cells. Since single cells only were collected for analysis, such cells are likely to have undergone DNA synthesis or even mitosis, but failed to undergo cytokinesis. Inspection of p53as(+) cells grown on coverslips revealed that most (approximately 85%) contained two or more nuclei (data not shown), supporting the conclusion that these carcinoma cells continued to synthesize DNA and undergo Karyokinesis (nuclear division) but failed to undergo cell division. Nontransformed 291 cells, cultured under conditions favoring proliferation (LC), were treated similarly for comparison with carcinoma cells. As shown in Figs. 10A - 10F, untreated cells were primarily in the G2/M stage. In response to actinomycin D, the distribution changed in favor of G0/G1, suggesting that both p53as protein and p53 protein reactive with PAb421 or PAb246 contribute to G1 arrest of normal cells exposed to DNA damage. In contrast to carcinoma cells, a population of 291 cells positive for anti-p53as reactivity alone was observed (Figs. 10A - 10F). These showed a similar cell cycle distribution to cells labeled with anti-p53as and PAb421 or PAb246. Unlike the carcinoma cells, the p53as(+) 291 cells observed on coverslips were generally mononucleated.

The percentage distribution by cell cycle stage of nontransformed 291 and 291.05RAT carcinoma cells heated with actinomycin D or solvent controls are presented in Table 2. The preferential association of p53as antigen activity with G2/M and >G2/M, the association of p53 protein (reactive PAb421 and PAb246) with G0/G1 and the response to actinomycin D were consistent among a total of 3 independent experiments per cell type. In nontransformed 291 cells, actinomycin D increased the percentage of cells expressing immunodetectable p53as and p53(PAb421 and PAb246) by approximately 4-fold and resulted in preferential accumulation of cells in the G1 phase of the cell cycle compared to solvent controls. In contrast, the 291.05RAT tumor cells showed little difference up the percentage of cells in G1 in response to actinomycin D treatment, suggesting that the p53 protein in these cells was less capable of causing G1 arrest, even though the percentages of cells positive for PAb421 and PAb246 were elevated.

### Human p53as peptide

Human p53as protein is defined herein as the human p53 protein 1) which is generated from a human p53 transcript by reverse transcriptase (RT)/polymerase chain reaction (PCR) (as described below) which is itself generated by alternative splicing of a region of intron 10 of the human p53 gene, and 2) which contains carboxyl terminal amino acids distinct from those of the major human p53 protein. The p53 transcript may be detectable in human cells or may be synthesized. Antibodies selective for human p53as peptide would permit verification of the presence of the p53as in human cells.

Prior to the present invention, no human p53as protein in normal cells (alternatively spliced at the carboxyl terminus, analogous to mouse p53as) has been reported or suggested. The mouse and human p53 cDNA sequences are 81% identical and have functional domains in common. There are three lines of evidence pointing to the existence of naturally occuring human p53as. First, two PCR products have been amplified by RT/PCR from human cDNA using primers which span intron 10 created from mouse exon 10 and exon 11 sequences. Second, two p53 proteins are detectable by molecular weight differences in western immunoblots or immunoprecipitations using polyclonal antibodies to human p53, for example, by Gupta et al. (Proc. Natl. Acad. Sci. 90: 2817-2921, 1993) who used antibody CM-1 and protein from Hodgkins disease tumor cells. This has been attributed to either distinct phosphorylation states or a polymorphism at amino acid 72. CM-1 is expected to react with multiple regions on the p53 gene and thus would be expected to react with both human p53 and p53as proteins. Thus the presence of human p53as could account for the data in the literature demonstrating two p53 proteins distinguishable by molecular weight. Third, human intron 10 encodes a peptide which has a motif (SPPC) similar to the last 4 amino acids of the mouse p53as (SPNC).

A peptide unique to human p53as may be identified as follows:

Primers are constructed which are used to amplify by polymerase chain reaction (PCR) a region of the human p53 cDNA including part of exon 10, all of intron 10 sequences retained in the alternatively spliced p53 mRNA and part of exon 11. Human cDNA is generated from cellular RNA (isolated by guanidinium/cesium chloride extraction) by RT/PCR. RT/PCR is carried out as follows: 5µg of human cell total RNA is combined with 1mM each of 4 deoxynucleotidetriphosphates, 5µg random hexamer primer (to make cDNA to all available mRNA), 5µl AMV reverse transcriptase (RT, 5 to 10 units per µl), 3.5 mM MgCl₂ (or as optimized), 2.5µl Rnasin 5µl PCR buffer (Perkin Elmer; without Mg²⁺) and depc-treated water to adjust the volume to 50µl. Reaction is allowed to proceed at 23°C for 10 minutes, 42°C for 1h and 95°C for 10 minutes then transferred to ice. An additional 0.2µl of RT is added and the reaction is repeated 1X. 1µl of the RT reaction product mix is used to provide the cDNA templates for human p53 and p53as C-terminal regions for amplification by PCR. PCR is optimized to obtain efficient production of the specific product and minimize background. PCR is performed for 35 cycles of denaturation (95°C, 30 sec), annealing (60°C, 1 min) and extension (72°C, 3 min) in a DNA thermal cycler. Amplified fragments of human p53 and p53as C-terminal coding regions are desalted by centricon ultrafiltration, digested with the restriction enzymes appropriate to the synthetic primers (see example below) and isolated from low melting temperature agarose for cloning into pGEM3zf(+) (Promega) for the sense strand or pBluescript KS(+) (Stratagene) for the antisense strand and transfected into E. coli for production and sequencing as we have described (Han et al. supra). Human cells as the source of RNA include (but are not limited to) normal human epidermal keratinocytes and two clones (B and F2A) of squamous cell carcinoma line SCC-12. The PCR amplification product generated using the primers which span intron 10 include the major p53 transcript and p53as transcript(s). These are distinguished by differences in molecular weight and/or by sequencing of the amplified PCR products as has been demonstrated previously for mouse p53as transcripts (Han et al. (b) supra). Sequencing of the PCR products permits determination of the sequence of the protein encoded by human p53as RNA. The amino acid sequence of the human p53as protein is compared to that of the major human p53 protein to determine the unique sequence at the carboxyl terminal region of human p53 as protein.

An example of a primer set which spans intron 10 of the human p53 gene is: 5' primer/sense strand ATCGAAGCTTGAGATGTTCCGAGAGAGCTGAAT (within exon 10 beginning at nucleotide 17,593 of the genomic p53 sequence Genbank accession No. X54156, with additional nucleotides added to the 5' end, ATCG and restriction endonuclease site HindIII to facilitate cloning and sequencing - underlined (Sequence ID # 3)) and 3' primer antisense strand ATCGTCTAGAGCTTCTGACGCACACCTATTG (within exon 11 beginning at nucleotide 18,794 in the 5' to 3' direction to nucleotide 18,774, with ATCG and XbaI restriction endonuclease site added - underlined (Sequence ID # 4)).

Alternatively, human p53 DNA or RNA may be restricted to remove the p53 negative regulatory domain. A unique sequence may then be grafted to restricted p53 which will give rise to a p53as protein to which a specific antibody may be raised.

### Polyclonal antibody specific for mouse p53as protein

Polyclonal antibody to mouse p53as unique peptide noted above has been raised in rabbits, its high titer has been determined by enzyme linked immunosorbent assay (ELISA), its specificity for p53 protein has been determined by immunoprecipitation from mouse cells, western immunoblotting of anti-p53 precipitable protein to a polyclonal antibody to p53 (CM5, reactive with epitopes shared by p53 and p53as proteins), ability of the peptide to competitively block reactivity in cells and in western immunoblots, and the ability of the p53as peptide to block blinding of p53as antibody but not block the binding other p53 antibodies (PAb421 and PAb246) which bind to epitopes distinct from the unique region of p53as.

The polyclonal anti-peptide antibody is produced in rabbits as described in General Procedures below.

### Monoclonal antibody specific for mouse p53as protein

Hybridoma cell lines have been produced by fusion of spleen cells from BALBc mice immunized with mouse p53as peptide. The procedure is found in General Procedures below.

The monoclonal antibodies from each hybridoma cell line producing specific antibody as determined by ELISA is to be tested for reactivity with mouse cellular p53as by immunoprecipitation, western immunoblotting and immunofluorescence as described for polyclonal antibody to mouse p53as above. Specificity is determined by competition with mouse p53as peptide.

### Polyclonal antibody specific for human p53as protein

A peptide unique to human p53as, determined as described above, will be synthesized and used to immunize rabbits following the procedures used to generate polyclonal antibody to mouse p53as, described in the manuscript provided and in General Procedures. An example of such a peptide selected based upon similarities with mouse p53as unique peptide is the following 20 amino acid peptide encoded by human intron 10 sequences: REKGHRPSHSCDVISPPCFC (Sequence ID # 5).

### Monoclonal antibody specific for human p53as protein

A peptide unique to human p53as, determined as described above, will be synthesized and used to immunize mice following the procedures used for polyclonal antibody to mouse p53as described above, and in General Procedures below.

Note that the species specificity of each antibody will be determined; that is, for example, testing of whether an antibody generated to mouse p53as peptide also binds human p53as and whether antibody to human p53as binds mouse p53as protein will be performed.

### General procedures for immunizing mice with synthetic p53as peptides (mouse or human) - monoclonal antibody production

The procedures to be used are based on standard procedures for generating monoclonal antibodies.

The p53as peptide of mouse or human origin is stored protected from light and oxygen until use. It is reconstituted just prior to injection. Unmodified peptide is used as immunogen initially because this was successful in the generation of polyclonal antibodies to mouse p53as protein. Alternatives which will be used if necessary to improve immunization include conjugation to another protein (for example, ovalbumin), or use of full length p53as protein generated in insect cells using a baculovirus vector system. Such vectors containing p53as cDNA have already been made in this laboratory for production of mouse p53as protein.

For each mouse, 250 µl (30 to 50 µg of peptide) is emulsified with an equal volume of Freund's complete adjuvant.

The emulsion is injected into BALB/c female mice (weighing approximately 20g each) intradermally at multiple sites along the dorsum and intraperitoneally. (If necessary to improve the immunization response, an alternative mouse strain will be used.)

Four weeks later, boosting of the mice with an intraperitoneal injection of 100 µl (20 µg peptide) mixed with Freund's incomplete adjuvant is performed.

Two weeks later, serum is tested for antibody titer by ELISA as per manuscript provided.

Three days before the fusion, the best responder is reboosted with an intravenous injection of 100 µl (20 µg peptide) without adjuvant.

### Cell Fusion

### Preparing myeloma cells for fusions

Myeloma cells are thawed from liquid nitrogen and placed in culture one week prior to the fusion. The cells are grown in order to reach a cell density of 5 x 10⁵ cells/ml one day before the fusion. On the morning of the fusion, 10 ml of cultured cells are diluted with an equal volume of CMEM.

### Complete Media Preparation (CMEM):

- 0.5 ml: gentamicin sulfate
- 5.0 ml: Pyruvic acid stock solution
- 5.0 ml: Hypoxanthine stock solution
- 5.0 ml: Thymidine stock solution
- 5.0 ml: Oxaloacetate stock solution
- 5.0 ml: Penicillin G stock solution
- 5.0 ml: Bovine insulin stock solution
- 50 ml: NCTC 109 (MA Bioproducts)
- 100 ml: Fetal Bovine Serum (heat inactivated)

The above solutions are added to a sterile 500 ml bottle and volume is adjusted to 500 ml with Dulbecco's MEM (with L-glutamine, with D-Glucose at 4500 mg/L, without Sodium Pyruvate, Gibco). The medium is sterilized by 0.2 micron filtration and tested for contamination by incubate overnight at 37°C. CMEM is stored at 4°C and used within 2 weeks.

### Preparing splenocytes for fusions

The mouse is sacrificed and the spleen is aseptically removed. Contaminating tissues are dissected and discarded.

The spleen is ground on a stainless steel mesh to release the cells.

The splenocytes are washed twice with 10 ml of medium without serum and the cells counted.

### Cell fusion

Myeloma cells are washed once and resuspended in medium without serum.

The myeloma cells and splenocytes (1:10) are combined in medium without serum. These cells are centrifuged together at 800 g for 5 min.

The supernatant is removed. 50% PEG 1500 is added to the cell pellet slowly over 1 min while resuspending the cells by stirring with the end of the pipet. Stirring is continued for an additional minute. Then 1 ml medium without serum is added to the cell suspension over the next minute. Finally, 9 ml medium is added over 2 min with stirring. The cells are centrifuged at 400 g for 5 min.

The supernatant is removed and the cells resuspended in 30 ml HAT media.

### HAT Media Preparation:

HAT medium is prepared the same as CMEM but 1.0 ml aminopterin stock solution and 1.0 ml glycine stock solution are added before bringing media to a total volume of 100 ml.

100 ml of cells are dispensed into the wells of 96-well plates. The plates are incubated in a 5% CO₂ atmosphere.

### Single-cell Cloning

### Screening positive clones by ELISA.

About 50 µl of culture supernatant are placed in the wells of another 96-well microtiter plate that has been coated with p53as synthetic peptide appropriate to the antibody (mouse or human p53as peptide). Positive clones are detected by ELISA as presented in the manuscript attached.

### Preserving positive clones.

After a positive well has been identified, the cells are transferred from the 96-well plate to the well of the 24-well plate containing the same medium. After the 24-well plate culture becomes dense, it is transferred to 100-mm dish. Freeze the cells at the 100-mm dish stage.

### Limiting dilution

On the day before cloning, a spleen cell suspension is prepared according to the procedure described in the fusion technique above. 10³ spleen cells per well are plated into a 96-well plate (using one drop per well). A minimum of 10⁵ proliferative hybridoma cells from a 25 cm² flask are used for cloning.

The hybridoma cells are subcultured 24-48 hours before cloning by diluting an actively growing culture 1:1 with fresh media. Cells in mid-log phase are used for the limiting dilution.

Cell number is adjusted to 10⁵ viable sells per ml (cell viability of >70%).

Serial 10-fold dilutions are made (e.g. 10⁴, 10³ per ml.)

In a 50 ml tube, 0.30 ml of the 10³ hybridoma cells is added per ml and 29.7 ml of CMEM.

One drop from a 2.0 ml (50 µl) pipette of the cell suspension is added to each well of the 96 well plates with the spleen cells prepared the day before cloning.

Cultures are observed every 2 to 3 days and wells with a single cell clone are marked.

Clones are assayed for anti-p53 as activity by ELISA when they cover 25% of the well. Positive clones are transferred to 24 well plates, to 100-mm dishes, then hybridoma cells are cryopreserved in Nunc cryotubes.

### Ascites Production

8 week old BALB/c mice are injected with 0.4 ml of pristane intraperitoneally.

After two weeks, 0.5 ml cells (10⁵ cells) in mid-log phase are injected into each pristane-treated mouse.

After 2-3 weeks, the mice are sacrificed and the ascitic fluid harvested.

Ascites is centrifuged at 1000 g for 10 min, the middle layer is collected and kept at -20°C.

Monoclonal antibodies are tested by ELISA, indirect immunofluorescence, immunoprecipitation and western immunoblotting for specificity to p53as in appropriate cells (mouse or human). Ability of the antibody reactivity to be competitively blocked by the peptide used to generate it is tested.

### General procedures for immunizing rabbits with synthetic p53as peptides (mouse or human) - polyclonal antibody production

The procedures to be used are based on standard procedures for generating polyclonal antibodies.

The p53as peptide of mouse or human origin is stored protected from light and oxygen until use. It is reconstituted just prior to injection. As noted above for monoclonal antibody production, unmodified peptide is used as immunogen initially but, if necessary, conjugation to another protein will be done or full length p53as protein will be used. New Zealand white female rabbits (6-8 lb.) (4) are used for immunizations.

### Procedure

### Basal (pre-immune) serum is collected 1 wk. before immunization.

(day 0) 500 µg peptide per rabbit is freshly dissolved in phosphate buffered saline (PBS) & mixed with Freund's complete adjuvant (total vol. 2 ml or less) and used as antigen. Immunization is by intradermal injection at multiple sites (at least 10 sites) along the dorsum and concurrent intramuscular injection of Pertussis vaccine.
(day 21) 250 µg peptide/rabbit is freshly dissolved in PBS & mixed with Freund's incomplete adjuvant (tot. vol. 1 ml or less). Injections are intradermal on the back (at least 10 sites).
(day 28) Bleed for serum. Test anti-p53as antibody titers by ELISA
(day 35) Immunization with 250 µg peptide/rabbit as day 21.
(day 42) Test bleed for serum.
(day 49) Immunization with 250 µg peptide/rabbit as day 21 & 35.
(day 56, day 63) Bleed for serum.
(day 77) Immunization with 250 µg peptide/rabbit as above.
(day 84) Bleed for serum on the following weekly schedule: rest, boost as necessary to maintain antibody titer (250 µg peptide/rabbit) bleed each week for 3 weeks, rest 1 week, repeat with boosting as necessary to maintain titer.

### Affinity purification of antibody

Polyclonal antibody is affinity purified by complexing with the appropriate peptide (human or mouse p53as) coupled to an AminoLink column (see manuscript provided) through amino groups or a column in which peptide is bound through cysteine. Approximately 7 mg of peptide is required for binding to the column and antibody from approximately 25 ml of serum is purified per run. The column is reconstituted according to manufacturer's directions and reused. Affinity purified antibody is tested by ELISA, indirect immunofluorescence, immunoprecipitation and western immunoblotting for specificity to p53as. Ability of the antibody reactivity to be competitively blocked by the peptide used to generate it is tested.

As evidenced above, p53as, a physiological variant of the tumor suppressor protein p53, has been detected in mammalian epidermal cells containing the wt. p53 gene. The fact that endogenous wild type p53as protein has not been detected in cells before may be due to a number of factors including the previous failure to recognize its possible existence, its low abundance and lack of reactivity with anti-p53 monoclonal antibody PAb421. It was previously shown that the wt. p53as mRNA is present in normal mouse tissues and in cultured mouse fibroblasts at 25 to 33% of the major p53 RNA form (Han et al., (b), Alternatively spliced p53 RNA in transformed and normal cells of different tissue types, Nucleic Acids Res., 20(8), 1979-1981). In accordance with the present invention, it has been demonstrated that the p53as protein exists in cells, that it is nuclear in location in normal epidermal and carcinoma cells, is differentially expressed in proliferating compared to differentiating normal cells and is inducible along with PAb421- and PAb246-reactive p53 by the DNA damaging agent actinomycin D.

The presence of cells with greater than the G2/M content in acetone controls suggests that these are a physiological component of the epidermal cell populations. The higher ratio of p53as to other p53 antibody reactivities in cells cultured under LC conditions suggests that p53as is preferentially expressed in proliferating populations. Yet, the distribution of p53as-positive cells in G2/M and >G2/M appears more likely to reflect a growth arrest or maturation pathway. Epidermal cell populations are composed of proliferating and differentiating or growth-arrested cells, reflecting the balance of growth and differentiation strictly maintained in epidermis. Even under conditions favoring proliferation (LC conditions, see Experimental Procedures) basal (proliferation-associated) cell markers are lost with time after plating and differentiating cells increase as a percentage of the total population (Kulesz-Martin et al., (1989), "Pemphigoid, pemphigus and desmoplakin as antigenic markers of differentiation in normal and tumorigenic mouse keratinocyte lines", Cell Tissue Kinet, 22, 279-290). Mouse keratinocytes exhibit a bimodal DNA content, containing stable populations near diploid and near tetraploid 3 to 19 days after establishment in primary culture (Kulesz-Martin et al. (1983), "Properties of carcinogen altered mouse epidermal cells resistant to calcium-induced terminal differentiation", Carcinogen, 4, 1367-1377). While the 291 cells are sub-tetraploid (Kulesz-Martin et al., (1985) "Mouse cell clones for improved quantitation of carcinogen-induced altered differentiation", "Carcinogenesis" 6, 1245-1254) they appear to retain the capacity to generate a subpopulation of cells with doubled DNA content. Davies et al. (1993), "Antioxidants can delay liver cell maturation which in turn affects γ-glutamyltranspeptidase expression", Carcinogen, 14, 47-52, have discussed the changes in ploidy from 2N to 4N and 8N which occur by mitosis without cytokinesis and accompany maturation of normal liver cells. They note that after partial hepatectomy, regenerating liver contains increased numbers of cycling cells and binucleated tetraploid cells which undergo DNA synthesis and amitotic cytokinesis, resulting in mononucleated tetraploid cells. The presence of cells with >G2/M DNA content in populations of epidermal cells could reflect progress along a maturation pathway. The increase in percentage of p53as(+) 291 nontransformed cells in response to actinomycin D suggests that p53as protein cooperates with p53 detectable by PAb421 and PAb246 in the DNA damage induced G1 arrest. However, the preferential association of p53as protein immunoreactivity with G2/M occurs in control and in actinomycin D-treated cells, suggesting that it reflects a physiological activity of p53as protein in the G2 state rather than a consequence of treatment. It is intriguing that actinomycin D and γ-ray treatment induces G1 and G2 arrest, but only G1 arrest was associated with a rise in PAb421 activity (Kastan et al., supra). One could speculate that p53as protein has a role in G2/M arrest in response to DNA damage. In contrast, regardless of increased percentage of p53-positive cells, no actinomycin D-dependent changes in cell cycle distribution occurred in carcinoma cells, suggesting that carcinoma cells are defective in the ability to undergo cell cycle arrest.

Additional inferences about the functional properties of p53as protein can be made based on the studies of others. Changes at the carboxyl terminus of p53 protein engineered by site-directed mutagenesis or deletion mapping have been shown to have dramatic effects on p53 structure and function (Hupp et al., supra). Sturzbecher et al., supra, demonstrated that loss of C-terminal basic residues permitted dimer formation of p53 protein but not tetramers, while Hainaut et al., (1992), "Interaction of heat-shock protein 70 with p53 translated *in vitro* evidence for interaction with dimeric p53 and for a role in the regulation of p53 conformation", EMBO J. 11, 3513-3520 showed that deletion of 25 C-terminal amino acids of p53, similarly, resulted in dimers but not higher order complexes. Since p53 protein is thought to bind to DNA as a tetramer (Bargonetti et al., (1992), "Site-specific binding of wild-type p53 to cellular DNA is inhibited by SV40 T antigen and mutant p53", Genes & Dev. 6, 1886-1898; and Stenger et al., (1992), "Formation of stable p53 homotetramers and multiples of tetramers", Mol. Carcinogen, 5, 102-106), restriction of p53as to dimer formation may influence its interactions with DNA.

Properties of the mutated form of alternatively spliced p53 protein translated *in vitro* from the M-8 cDNA clone (Arai et al., supra) have been reported. Hainaut et al. supra, observed that, following *in vitro* translation, the mutant alternatively spliced p53 protein encoded by M-8 formed monomers and dimers, but not tetramers. Like the mutant M-8 protein, wt. p53as has lost the basic amino acids of the C-terminus but retains the acidic amino acids shown to permit dimer formation. These results support the idea that wt. p53as may have distinct properties from the major p53 protein form. However, the M-8 p53 cDNA sequence has a nucleotide substitution at nt 395 resulting in a change from cysteine-132 to phenylalanine. Eliyahu et al. (1990), "Meth a fibrosarcoma cells express two transforming mutant p53 species", Oncogene 3, 313-321), reported that plasmids containing the M-8 p53 cDNA had transforming activity in transfected cells. Mutations within this region of p53 without alternative splicing are defective in tumor, suppressor function and DNA binding (Eliyahu et al., supra; Finlay et al., (1989) "The p53 proto-oncogene can act as a suppressor of transformation", Cell, 57, 1083-1093; and Vogelstein et al., (1992) supra). Thus the M-8 protein has a mutation which affects its function apart from the C-terminal changes due to alternative splicing. p53 protein translated from cDNA clone M-8 does not react with PAb248 antibody (which, like PAb246 is wt. conformation-specific) or, due to loss of the C-terminal epitope, PAb421 (Wolf et al., (1985) supra). Since the wt. p53as has a distinct conformation compared to the mutated M-8 protein, functional properties such as DNA binding cannot be predicted from studies of M-8 protein and must be tested directly.

In addition to alterations in the oligomerization domain predicted from the above studies, p53as has lost the casein kinase II phosphorylation/5.8s rRNA binding site located at serine 389. Loss of the phosphorylation site at serine-389 by mutation negates p53 anti-proliferative activity (Milne et al., (1992), "Mutation of the casein kinase II phosphorylation site abolishes the anti-proliferative activity of p53", Nucleic Acids Res. 20, 5565-5570; Bischoff et al., (1992), "Human p53 inhibits growth in Schizosaccharomyces pombe", Mol. and Cell. Biol. 12, 405-411; Nigro et al., (1992), "Human p53 and CDC2Hs genes combine to inhibit the proliferation of Saccharomyces cerevisiae", Mol. and Cell. Biol. 12, 1357-1365). Hupp et al., supra, have shown that factors acting at the C-terminus are important for activation of the DNA binding capacity of wt. p53, including: phosphorylation at the conserved C-terminal serine, PAb421 antibody binding to its carboxyl terminal epitope, and proteolysis or engineered loss of the last 30 carboxyl terminal amino acids. In addition, regions of the p53 protein which mediate binding to other cellular proteins such as heat shock protein (Hainaut et al., supra) or mdm2 (Momand et al. (1992), "The *mdm*-2 oncogene product forms a complex with the p53 protein and inhibits p53-mediated transactivation", Cell 69, 1237-1245), conceivably could be directly or indirectly altered.

The differential expression of p53 and p53as protein immunoreactivities during the cell cycle suggest that each has a distinct function. Perhaps p53 may prove to be within the class of transcription factors which generate two functionally distinct proteins by alternative splicing (Foulkes et al., (1992), "More is better: activators and repressors from the same gene", Cell 68, 411-414). For example, in the case of mTFE3 factor (which regulates immunoglobulin transcription by binding to promoter and enhancer regions), there is a longer and a shorter protein form. In the shorter form amino acids predicted to form an amphipathic helix are absent and transcriptional activation activity is affected. The ability of such factors to heterodimerize amplifies the possibilities for regulation of expression of target genes.

It will be important to determine the activity of wt. p53as in tumor suppression. While originally thought to be an oncogene because initial clones of p53 harbored mutations, cloning of wt. p53 lead to the recognition of its role as a tumor suppressor gene (Finlay et al., supra; and Eliyahu et al., supra). The capacity of mutant p53 protein to drive wt. p53 into the mutant conformation uncovered its potential as a dominant negative transforming gene (Milner, 1984, supra.; and Milner et al., (a) (1991), "Cotranslation of activated mutant p53 with wild type drives the wild-type p53 protein into the mutant conformation", Cell 65, 765-774). Yet mutant p53 itself has transforming activity in cells which have no wt. p53, suggesting direct activities of p53 in the regulation of proliferation (Wolf et al., (1984) , "Reconstitution of p53 expression in a nonproducer Ab-MuLV-transformed cell line by transfection of a functional p53 gene", Cell 38, 119-126). Milner (b) (1991), "The role of p53 in the normal control of cell proliferation", Current Opinion in Cell Biology 3, 282-286, has proposed that p53 has positive and negative functions in cell cycle regulation, dependent upon p53 conformation. One could speculate that positive or negative functions of p53 in regulation of cell cycle progression or induction of apoptosis may reside in the relative expression of different physiological variants of p53 generated by alternative splicing. Studies to compare differential functional activity of the wt. p53 and p53as in DNA binding, transcriptional activation, cellular transformation, cell cycle arrest and apoptosis will be necessary to test these possibilities.

### Experimental Procedures

### Cells

The strain 291 was derived from neonatal BALB/cROS mouse epidermis (West Seneca Laboratory, Roswell Park Cancer Institute) and is "normal" with respect to differentiation and morphology *in vitro* and *in vivo* (Kulesz-Martin et al., (1985) supra; Kulesz-Martin et al., (1991), "Tumor progression of murine epidermal cells after treatment *in vitro* with 12-0-tetradecanoylphorbol-13-acetate or retinoic acid", Cancer Research 51, 4701-4706; Schneider et al., (1993), "7, 12-dimethylbenz[α]anthracene-induced mouse keratinocyte transformation without Harvey ras protooncogene mutations", J. Invest. Dermatology, in press). The cells were grown in Eagle's minimum essential medium with Earle's salts without CaCl₂ (GIBCO, Grand Island, NY), supplemented with 5% (v/v) fetal calf serum treated with chelex-100 resin (Bio-Rad, Rockville Center, NY) to reduce CA²⁺ concentration, non-essential amino acids, 10% (v/v) mouse dermal fibroblast conditioned medium, 10ng/ml EGF (UBI, Lake Placid, NY), 1% (v/v) antibiotic-antimycotic (100 µ/ml Penicillin, 100 µ/ml Streptomycin sulfate and 0.25 µg/ml Amphotericin B Solution, GIBCO, Grand Island, NY) and 0.02-0.04 mM Ca²⁺ (designated as LC). Tumor cell derivatives of 291 (291.03RAT and 291.05RAT) were isolated from squamous cell carcinomas following exposure of 291 cells to 7,12-dimethylbenz[α]anthracene *in vitro* as described (Kulesz-Martin et al., (1986), "Retinoic acid enhancement of an early step in the transformation of mouse epidermal cells *in vitro*", Carcinogenesis 7, 1425-1429; Kulesz-Martin et al., (1991) supra; Kulesz-Martin et al., (1983) supra. Tumor cells were grown in minimum essential medium as above except without conditioning or EGF and with native fetal calf serum and 1.4 mM Ca²⁺ (designated as HC). Clone 119 was derived by transfection of carcinoma 291.03RAT with a plasmid containing a genomic clone of mutant p53 (pmMTval135-23), obtained from Dr. Moshe Oren. It expresses predominantly wt. p53 conformation at 37°C (unpublished results).

### Antibodies

Mouse monoclonal antibodies to p53 were PAb421, PAb240 and PAb246 (Oncogene Science, Uniondale, NY). Isotype IgG₂ₐ (PAb421 and PAb240) and IgG₁ (PAb246, Becton/Dickinson, Mountain View, CA) were used as sera controls. Rabbit polyclonal antibody CM5 was a gift from Dr. David Lane. Anti-peptide antibody to the terminal 17 amino acids unique to p53as (see Fig. 1B) was generated as follows: The 17 amino acid peptide to p53as was synthesized by the RPCI Biopolymer Facility and determined to be 90 to 95% pure by HPLC and mass spectroscopy and accurate by amino acid sequencing. Following collection of pre-immune serum, New Zealand White female rabbits were immunized by intradermal injection of 500 µg peptide plus Freund's complete adjuvant (FCA) at multiple sites, concurrent with intramuscular injection of Pertussis vaccine (RPCI Springville Laboratories). After 3 weeks, an additional 250 µg of peptide was administered with Freund's incomplete adjuvant (FIA) at weekly intervals for 3 weeks. The p53as anti-peptide serum was affinity-purified by coupling 4.4 mg p53as peptide to an AminoLink column (Pierce, Rockford, IL) according to manufacturers instructions. Ammonium sulfate (40%)-precipitated pre-immune serum was used as a control. Competition assays were performed by incubation of antibodies (1:1 ratio by weight) with p53as peptide or an unrelated peptide (sequence: GRNDCIIDKIRRKNCD (Sequence ID # 6)) for 2h at room temperature prior to the immunoreaction with cells or peptide in ELISA assays.

### Enzyme-linked immunosorbent assay (ELISA)

Nunc-Immuno MaxiSorb 96 well plates (Nunc, Denmark) were coated with 50 ng/well p53as peptide in 15 mM sodium carbonate buffer, pH 9.6. After blocking with 2% BSA (KPL, Gaithersburg, MD) in PBS at 37°C for 1h, anti-p53 monoclonal antibodies, anti-p53as antibody (affinity-purified to peptide) or pre-immune serum control (pre-I) were diluted 1/50 to 1/640,000 and added to the wells in 100 µ volume. Secondary antibody was peroxidase-conjugated goat anti-rabbit immunoglobulin (DAKO, Carpinteria, CA) at 1/1000. TMB peroxidase substrate system solution (KPL, Gaithersburg, MD) was added and color development was terminated after 4 minutes using 4M H₂SO₄. Absorbence at 450nm was detected using a BioTek plate reader (Winooski, VT).

### Immunofluorescence

Cells were permeabilized with 100% cold EtOH, rehydrated in PAB (PBS + 0.1% Na-azide + 0.5% BSA) + 0.05% Tween 20 for 10 minutes, blocked with 5% normal goat serum (Vector Labs, Burlingame, CA) and then exposed to 10 µg/ml each of monoclonal anti-p53 antibodies PAb421, 240 or 246 or control isotype sera IgG₂ₐ or IgG₁ overnight at 4°C. Secondary antibody for monoclonal antibodies was a 1/300 dilution of fluorescein isothiocyanate (FITC)-conjugated goat anti-mouse immunoglobulin (FisherBiotech, Pittsburgh, PA). Affinity purified rabbit polyclonal anti-p53as or ammonium sulfate-fractionated pre-immune serum as control was used at 7 µg/ml followed by 1/300 Texas Red-conjugated goat anti-rabbit immunoglobulin (Oncogene Science). FluorSave aqueous mountant (Calbiochem, LaJolla, CA) was used to attach coverslips to slides. The fluorescence was viewed using a Nikon Labophot microscope equipped with epiilluminescence. Photomicrographs were taken the a Nikon UFX-IIA automatic camera system.

### Immunoprecipitation

Preconfluent cultured cells (approximately 5-10 x 10⁶ cells/100mm petri dish) were incubated with 200 µCi of L-[³⁵S]methionine (1120 Ci/mmol) for 4h at 37°C in methionine-free minimum essential medium containing 2% (v/v) dialyzed calf serum. Labeled cells were lysed in buffer containing 1% (v/v) nonidet P-40, 150mM NaCl, 50mM Tris pH 8, 1mM phenylmethylsulfonyl fluoride for 30 minutes at 4°C and centrifuged at 10,000 x g for 10 minutes. The supernatant was precleared with formalin-fixed *Staphylococcus aureus* cells (Immunoprecipitin, BRL, Gaithersburg, MD) or with protein A-Sepharose (Pharmacia, Piscataway, NJ). Lysate volumes corresponding to equal amount of radioactivity (2 x 10⁷ cpm) were incubated in NET/Gel buffet (150mM NaCl, 5mM EDTA, 50mM Tris pH 7.4, 0.05% NP-40, 0.025 NaN₃ and 0.25% gelatin) for 16h at 4°C with antibodies to murine p53 or isotype or serum controls. Immune complexes were precipitated with Immunoprecipitin or 5mg of protein-A SepharoseCL-4B (Pharmacia) for 2h at 4°C, centrifuged at 10,000 x g for 10 minutes. The pellets were washed with NET/Gel buffer and eluted in loading buffer (2% (w/v) SDS, 10% (v/v) glycerol, 125 mM Tris-Cl, pH 6.8, 0.001% (w/v) bromophenol blue) by heating at 85°C for 5 to 15 minutes and centrifugation at 10,000 x g for 10 minutes. Supernatants were loaded on a denaturing polyacrylamide gel composed of 4% stacking gel (125mM Tris-Cl, pH 6.8, 0.1% (w/v) SDS) and 10% separating gel (375mM Tris-Cl, pH 8.8, 0.1% (w/v) SDS), and subjected to electrophoresis at 35mA in running buffer (125mM Tris-Cl, pH 8.3, 192mM glycine, 0.1% (w/v) SDS). Gels were fixed in 7.5% (v/v) acetic acid/25% (v/v) methanol, soaked in enhancer solution (NEN, Boston, MA) and dried prior to exposure to XAR film (Kodak, Rochester, NY) at -80°C with intensifying screens.

### Treatment with Actinomycin D

Cells on coverslips were treated with 0.25nM or 0.5nM actinomycin D (Sigma, St. Louis, MO) or 0.2% acetone for 48 h, beginning 24h after plating and stained by indirect immunofluorescence as described above. For flow cytometry or isolation of cellular RNA, approximately 2 to 4 x 10⁶ cells/cm² were seeded in 150mm plates, grown to 70% confluence, treated with 0.5mM actinomycin D for 48h before harvest.

### Northern blot analysis

RNA was isolated from cells approximately 70 to 100% confluent by guanidinium/cesium chloride extraction and dissolved in diethylpyrocarbonate-treated water for northern blot analysis as described previously (Han et al. (1990), "Altered levels of endogenous retrovirus-like sequence (VL30) RNA during mouse epidermal cell carcinogenesis", Mol. Carcinogenesis 3:75-82). A 500 base pair PstI fragment of p53-422 was used for p53 detection (Oren et al. (1983), "Molecular cloning of a cDNA specific for the murine p53 cellular tumor antigen", Proc. Natl. Acad. Sci. USA, 80, 56-59) and as 840bp EcoRI-SalI fragment of pA6 was used for 7S RNA detection as a control for RNA loading (Balmain et al., (1982), "Cloning and characterization of the abundant cytoplasmic 7S RNA from mouse cells", Nucleic Acids. Res., 10, 4259-4277). Probes were labeled with [α-32P]dCTP by the random primer method using a multiprime labeling kit (Amersham, Arlington Heights, IL). 32P-labeled probe was used at a final concentration of 1 to 2 x 10⁶cpm/ml. Differences in p53 RNA abundance were quantitated by densitometry of exposed films (Fastscan computing densitometer, Molecular Dynamics, Sunnyvale, CA) after adjustment for 7S RNA.

### Flow cytometry

Cells permeabilized in suspension with 100% cold ethanol were exposed to anti-p53 antibodies as described for indirect immunofluorescence above except that the secondary reagent for anti-p53as was phycoerythrin (PE)-conjugated goat anti-rabbit IgM and IgG (FisherBiotech, Pittsburgh, PA). Hoechst 33342 (1µg/ml, bisbenzimide H, Calbiochem, La Jolla, CA) was added 1h prior to flow analysis for detection of DNA content. Analysis was performed on a FACSTAR⁺ dual 5 watt argon laser system (Becton Dickinson Immunocytometry Systems, San Jose, CA) with the primary laser lasing at 488nm at 200mW and the secondary laser lasing at 350nm at 50mW. PE and Hoechst emissions were passed through discretion filters with band widths of 575+/-13nm and 424+/-22nm, respectively. Flow cytometry data was acquired using Becton Dickinson standard acquisition software to exclude cell aggregates and debris and to collect single cell events only. Data was analyzed using Lysis II software (Becton Dickinson Immunocytometry Systems, San Jose, CA).

Table 1 shows response of 291.05RAT carcinoma cells to actinomycin D as detected by indirect immunofluorescence in situ. Two experiments (numbered) are shown. Cells were plated on coverslips, exposed to actinomycin D at the concentration indicated (nM) or solvent (0.2% acetone) for 48h, then stained with p53 antibodies. Estimates of positive cells as a percentage of total cells were made based on viewing all cells per slip (10 control, 8 to 10 treated slips per experiment for p53as; 4 control, 2 to 4 treated per experiment for PAb421; 2 control and 2 treated per experiment for PAb246, pre-immune and IgG controls. The range of percent positive cells is shown for 2 independent experiments.

Table 2 shows cell cycle distribution of mouse epidermal cells according to p53 antibody reactivities. Cells were exposed to 0.5 nM actinomycin D or solvent for 2 days, harvested and stained as described in Fig. 7 and Experimental Procedures. The data shown are the mean and Std. Dev. of percentages of cells positive for each antibody based on 2 x 10⁶ stained cells in 12 separate tubes (R4 negative cells or total cells), 6 tubes (anti-p53as) or 3 tubes each (PAb421 and PAb246). The results are representative of 2 separate experiments and 2 stainings of the same cell preparation. The percentage of cells in G0/G1 vs. G2/M and >G2/M were consistent among experiments within a cell type.

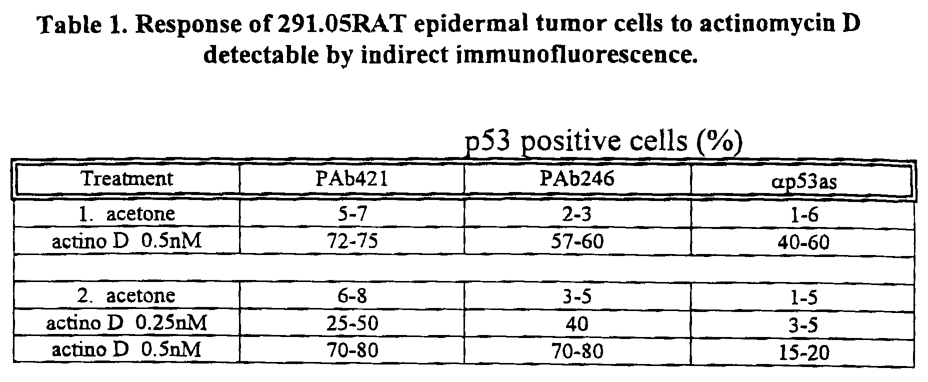

**Table 2**

| **Cell cycle distribution by flow cytometry of mouse epidermal cells according to p53 antibody reactivities.** | | | | | | |
|---|---|---|---|---|---|---|
| Cells | Treatment | Stage | (-)Cells | αp53as | PAb421 | PAb246 |
| 291LC | acetone | >G2/M | 0.6±0.2 | 33±6 | 4±0.6 | 0.3±0.2 |
| | | G2/M | 18±1 | 43±7 | 30±3 | 34±2 |
| | | S | 11±1 | 9±2 | 14±3 | 11±1 |
| | | G0/G1 | 70±1 | 15±2 | 52±1 | 48±1 |
| | | %total cells | 100±0.2 | 0.5±0.2 | 8±2 | 3±0.2 |
| | | | | | | |
| 291LC | actino D | >G2/M | 0.3±0.2 | 3±2 | 0.5±0.1 | 0.3±0.2 |
| | | G2/M | 9±2 | 30±2 | 10±1 | 11±1 |
| | | S | 8±0.9 | 10±1 | 8±0.4 | 9±0.5 |
| | | G0/G1 | 83±2 | 56±4 | 82±1 | 80±1 |
| | | %total cells | 100±0.6 | 2±0.8 | 36±3 | 12±1 |
| | | | | | | |
| 05RAT | acetone | >G2/M | 1±0.4 | 23±6 | 8±1 | 6±0.5 |
| | | G2/M | 19±2 | 30±5 | 32±4 | 29±1 |
| | | S | 22±2 | 25±5 | 24±7 | 19±3 |
| | | G0/G1 | 58±3 | 23±3 | 36±5 | 46±2 |
| | | %total cells | 97±4 | 1±0.4 | 2±0.6 | 10±2 |
| | | | | | | |
| 05RAT | actino D | >G2/M | 1±0.4 | 3±3 | 1±1 | 0.8±0.1 |
| | | G2/M | 15±3 | 40±5 | 27±5 | 26±1 |
| | | S | 20±2 | 28±6 | 24±5 | 25±1 |
| | | G0/G1 | 64±4 | 28±4 | 48±1 | 48±1 |
| | | %total cells | 93±9 | 1±0.4 | 10±0.1 | 23±1 |

### Materials and Methods for DNA binding studies

### Plasmids for in vitro transcription and translation of p53as and p53 proteins.

Plasmids containing the cDNA sequence unique to p53as are included in this invention. One such plasmid is pBSp53as which contains full length alternatively spliced p53 cDNA. pBSp53as was constructed from p53 cDNA beginning at nt -111 of the (where 1 is the first ATG encoding methionine) and ending at nt 1539, cloned into the EcoRI and BamHI sites of pBluescript SK under the control of a T3 phage promoter. The N-terminal fragment of wt p53 was amplified by reverse transcriptase/polymerase chain reaction (RT-PCR) from a mouse epidermal cell RNA template and the C-terminal fragment of p53as was amplified by PCR from plasmid p6.4 (which contains an alternatively spliced p53 cDNA; Han and Kulesz-Martin, Nucl. Acids Res. 20: 1979-81, 1992) using primers which contained a StuI restriction site at the 5' end of the sense primer (AGTCAGGCCTTAGAGTTAAAGGATGCCCATGCTACAGA) (Sequence ID # 7) and a BamHI site at the 5' end of the antisense primer. pBSp53 was made from pBSp53as by replacement of the StuI/BamHI C-terminal fragment of p53as cDNA with the StuI/BamHI segment of wild type p53 cDNA from plasmid pLSVNc51 (ref. Oren). In particular, cDNA for the N-terminus of p53 (nt -111 to 1090) was made using template RNA from 291 nontransformed epidermal cells by means of a reverse transcriptase reaction, amplified by PCR and cloned into the EcoRI and BamHI sites of pBluescript SK under the control of a T3 phage promoter to create plasmid pBSRS13. The primers used for PCR were: sense, AGTCGAATTCATTGGGACCATCCTGGCT (Sequence ID # 8), antisense, AGTCGGATCCTGGAGTGAGCCCTGCTGTCT (Sequence ID # 9). These primers contained an EcoRI restriction site at the 5' end of the sense primer and a BamHI site at the 5' end of the antisense primer (denoted by underlining). The C-terminal p53 cDNA (nt 1028 to 1539) was amplified by PCR from plasmid p6.4 (which contains an alternatively spliced p53 cDNA) using primers which contained a StuI restriction site at the 5' end of the sense primer (AGTCAGGCCTTAGAGTTAAAGGATGCCCATGCTACAGA (Sequence ID # 7)) and a BamHI site at the 5' end of the antisense primer (as in Han and Kulesz-Martin, Nucl. Acids Res. 20: 1979-81, 1992). The StuI to BamHI segment of this PCR reaction product was then ligated to the StuI and BamHI sites of plasmid pBSRS13 to create plasmid pBSp53as, containing a full length alternatively spliced p53 cDNA. To construct pBSp53, the StuI and BamHI fragment from wt p53 cDNA was substituted for the StuI and BamHI fragment of the p53as cDNA in pBSp53as.

### In vitro transcription and translation

10µg of plasmid pBSp53as or pBSp53 was linearized by BamHI and transcribed with 20 Units T3 RNA polymerase for immunoprecipitation studies and DNA binding studies. The *in vitro* translation was performed according to a standard protocol (Promega) by incubating 3µg RNA with 35µl of rabbit reticulocyte lysate. For immunoprecipitations, 4µl (40µCi) ³⁵S-methionine was used for labeling of proteins. For co-translation of p53as and p53, an equal amount of each RNA was incubated with rabbit reticulocyte lysate (Promega).

Immunoprecipitation was performed by incubating 5µl ³⁵S-labeled protein with 1µg PAb421, ApAs or PAb246 at 4°C overnight. Protein A-Sepharose 4B was then added with gentle mixing at 4°C for 2 hr. After centrifugation, the pellets were washed three times with Net-gel buffer. The pellets were suspended in 2x sample buffer containing 100mM DTT and separated by electrophoresis in a 7.5% SDS-polyacrylamide minigel. The gel was enhanced, dried and exposed overnight to Kodak X film. Two lysate reactions are shown, p53 or p53as RNA - see labels at top. Antibodies used to immunoprecipitate protein from each lysate reaction are indicated.

For the DNA binding assay, 3 µg of sense RNA for p53 or p53as was added to 35µl reticulocyte lysate and adjusted to a total volume of 50µl for translation *in vitro*. For cotranslations, half the amount of each RNA was used. An aliquot of 5µl of translation mixture (or 2.5µl each lysate for the mixing experiments) was incubated with 2µg poly[d(I-C)] and 30,000 cpm (approximately 1 ng) of ³²P-end-labeled DNA probe in 20µl DNA binding buffer (0.1% Triton x-100, 4% glycerol, 1mM EDTA, 5mMDTT, 20 mM Tris-HCl, pH 7.2, 80 mM NaCl) at 4°C for 20 min. Where indicated, 200 ng of antibody was included in the reaction. Reaction products were separated on a 4% neutral polyacrylamide gel in 0.5 X TBE buffer. The gel was dried and labeled binding complexes were visualized by radioautography.

### Results, p53as protein binding to DNA

As presented in the original patent application, we suggested that p53as protein would be active for binding to DNA. DNA binding of the major p53 form is considered essential for its function as a cell cycle control gene. DNA binding is required for its activity as a transcription factor which controls the expression of other genes. The interaction of p53 with other proteins is of intense interest in the scientific community because such p53-associated factors may control the activity of p53 by affecting its binding to DNA. The data presented herein demonstrates that p53as protein binds to DNA, and that p53as and p53 protein associate with each other, suggesting that p53as is a newly discovered p53-associated protein.

The p53as protein has lost basic amino acids but has retained acidic amino acids important for oligomerization. The sequence-specific DNA binding activity of p53as protein translated *in vitro*, separately, or cotranslated with p53 protein, was studied in an attempt to answer the following questions: 1) does p53as protein, like the major p53 form, have sequence-specific DNA binding activity? and 2) does p53as protein interact with p53, modulating its ability to form a complex with DNA?

To answer these questions, specific antibodies to p53 and to p53as proteins were used (Table 3). The specificity of the antibodies was tested by competition with the p53as peptide as reported in the original patent application. Further evidence for specificity of the p53as antibody is reactivity of anti-p53as with p53as protein translated *in vitro* but not with the major p53 protein. The commercially available antibody PAb421 which is specific for an epitope lacking in p53as reacted with p53 protein translated *in vitro* but not with p53as protein (Fig 12.). This ensured that these antibodies would not cross react with the two proteins in the DNA binding assays. Therefore, the ability of the anti-p53as antibody to shift a complex between *in vitro* translated p53as protein or p53 + p53as cotranslated protein clearly indicates that p53as protein must be present in the protein/DNA complex.

In order to answer the first question above, electrophoretic mobility shift assays were performed using a ³²P-labeled double-stranded oligonucleotide probe corresponding to the p53 binding sequence shown in Table 4. As shown in Fig. 12, p53as protein translated *in vitro* gave a strong signal representing a shift from free probe (dark signal at bottom of figure) to a higher molecular weight complex composed of protein and the labeled DNA probe. The p35as protein bound specifically to the p53 binding sequence, as shown by loss of the band with unlabeled competing DNA probe (wt) being included in the reaction but not with unlabeled oligonucleotide corresponding to the mutated p53 binding sequence (mut). (Note that the first weak band above the free probe is nonspecific). The identity of the shifted band as a complex containing p53as is shown by supershifting of the p53as/DNA complex by anti-p53as antibody (ApAs) but not by preimmune serum (Pre) or anti-p53 antibody PAb421. The supershift of p53as protein by ApAs resulted in two higher molecular weight bands. Further verification of the ApAs reactive protein as a product of the p53 gene is provided in Fig. 13 by loss of the signal in the presence of anti-p53 antibodies PAb246 and CM5, which react with both p53 and p53as proteins.

In contrast, the binding of the major p53 form to specific DNA binding sequences is inefficient and requires activation (Hupp, et al., 1992). Fig. 14 demonstrates that the major p53 form translated *in vitro* is not active for DNA binding but required activation by PAb421. Activated p53 bound to DNA and resulted in a supershift of a single high molecular weight complex.

Because p53 has been reported to self-associate to form oligomers, for example dimers and tetramers (Stenger et al., 1992), and because p53as has retained acidic amino acids but not basic amino acids important for dimer and tetramer formation, the DNA binding of p53 and p53as proteins, translated together or mixed after separate translation, was examined. As shown in Fig. 15, cotranslated p53 reduced the signal for DNA binding compared to p53as alone. This appears to be due to a direct association of p53 and p53as proteins because PAb421 antibody now resulted in a supershift of two bands rather than the one obtained with p53 alone.

In contrast to cotranslation of p53 and p53as proteins, mixture of lysates containing each protein translated individually did not show inactivation of p53as by p53 protein for DNA binding, and PAb421 supershifted only one band, suggesting that p53 and p53as must be translated together for association to occur (Fig. 16). This is consistent with the report that oligomerization between human p53 and mouse p53 occurs when they are cotranslated, but not when mixed (Milner and Medcalf, 1991).

In order to directly test the ability of p53 protein to form a complex with p53as protein, immunoprecipitation of cotranslated proteins was done using PAb421 and the immunoprecipitated proteins were resolved by denaturing gel electrophoresis, blotted and exposed to ApAs antibody. Proteins in complex with the protein immunoprecipitated by the specific antibody will also precipitate. Proteins not in complex will not. The lower band in the immunoblot shown in Fig. 17 is the p53as band. These results indicated that cotranslated p53as and p53 proteins contain complexes of p53 and p53as immunoprecipitable by PAb421 and blotted by ApAs. A much weaker signal is generated in mixtures of p53 and p53as proteins translated individually, indicating that for stable complexes to form the proteins must be cotranslated.

### Summary and Interpretations of DNA Binding Studies

p53as protein exhibited the antibody binding properties of wild type p53 protein, PAb246+, PAb240-, but lacked the C-terminal epitope reactive with PAb421. p53as protein translated *in vitro* was activated for binding to a p53 DNA binding sequence. The major p53 protein, in contrast, required activation for DNA binding (by monoclonal antibody PAb421). There appears to be a direct interaction between p53as and p53 proteins which influenced the composition of the DNA binding complex and the magnitude of DNA binding. Because cotranslated p53 protein inactivated p53as protein for DNA binding, and because two bands were super-shifted by PAb421 in lysates containing p53as and p53 cotranslated proteins, compared to one band in lysate containing p53 alone, these results could be explained by binding to DNA of p53as and p53/p53as heterooligomers, in addition to the binding of p53.

The significance of these findings is that they are consistent with a functional role for p53as protein in cells, which may, at least in part, be distinct from the function(s) of the major p53 protein form. Considering that vectors and plasmids containing the p53 gene are being tested for applications in gene therapy, and considering the results herein that p53as is active for binding to a p53 binding sequence and that p53as interacts with p53, resulting in altered DNA binding, plasmids and vectors for the expression of mouse and human p53as in cells and for uses in gene therapy in humans are claimed herein.

### Cellular function of p53as

In the original patent application the association of p53as antigen activity with cells distributed primarily in the G2 phase of the cell cycle was presented. This was an important clue that p53as might have a distinct function compared to p53 protein. In order to examine whether p53as protein might have a direct role in cell cycle arrest, a DNA sequence containing the entire coding sequence of p53as was constructed. This sequence is different from the p53 coding sequence, as presented in the original patent application, and is different from previously reported DNA constructs. Constructs containing the p53as cDNA were expressed in insect cells and in mammalian cells and effects on the cell cycle distribution of cells were evaluated.

### Materials and Methods for p53as expression studies

### Insect cells

Methods were per manufacturers instructions (Invitrogen) and materials and included linear AcMNPV DNA and transfer vector (e.g. pVL 1392, pVL 1393) and insect cell line S. frugiperda Sf9, propagated at 27°C in Grace's supplemented insect medium containing 10% fetal bovine serum (GIBCO) and 10µg/ml gentamycin sulfate. pVL1393BGB53 baculovirus vector containing wt p53 cDNA was constructed by inserting the BglII/BamHI fragment of pLSVNc51 (including the entire wt p53cDNA) into the pVL 1393 vector. pVL1393Asp baculovirus vector (containing p53as cDNA) was constructed by replacement of the StuI/BamHI C-terminal fragment of p53as cDNA (Han and Kulesz-Martin, Nucl. Acids Res. 1992) with the BamHI/StuI fragment of the pVL1393BGB53 vector (see above). To purify recombinant viruses, Sf9 cells were cotransfected with linear AcMNPV DNA and the transfer vector containing p53as and grown for 3 days. Recombinant viruses were identified by plaque assays or serial dilutions. Alternatively, p53 and p53as baculovirus constructions will be cotransfected with linearized (PharMingen) virus DNA which allows propagation of only recombinant virus. Virus stocks which resulted in p53as expression in insect cells (assayed by immunoblotting using anti-p53as antibody) were expanded and used to infect insect cells for the flow cytometry studies.

Baculovirus pVL1393Asp stock was added to insect cell cultures at 3 x 10⁶ cells/60 mm dish. After several (2 to 4) days, cells were harvested by trypsinization and stained with anti-p53as antibody and analyzed by flow cytometry as detailed in the original patent application.

### Mammalian cells

### Plasmids

Plasmids for expression of p53as in mammalian cells were constructed. An example is given of the full length p53as cDNA under the control of a metallothionein promoter. However, other promoters which may increase or decrease expression in given cell types, such as the cytomegalovirus promoter, will be used as appropriate. pmMTBGB containing the full length wild type p53 cDNA beginning at -67 nt (where nucleotide 1 is the first nucleotide of the first ATG codon) was constructed by replacement of the BgIII/BamHI fragments of plasmid pmMTval53cG (from M. Oren) with the BgIII/BamHI fragment of plasmid pLSVNc51 noted above. The pmMTAsp plasmid was constructed by firstly, replacement of the XhoI/BamHI fragment of pmMTBGB with the XboI/BamHI fragment of pVL1393Asp and secondly, introduction of a BamHI fragment from plasmid BCMGNeo (Karasuyama and Melchers, 1988) containing the splicing signals and polyA tail of the rabbit B-globin gene.

### Transfection

Mouse squamous cell carcinoma cells (291.05RAT) were plated in culture medium at 2 to 3 x 10⁶ per 60mm dish and transfected with plasmid pmMTAsp when 40 to 60% confluent using Lipofectin GIBCO BRL. 10µg of plasmid diluted in 100 µl ddH₂O was mixed with 30µl Lipofectin adjusted to 1 ml with serum-free culture medium, incubated with the cell cultures for 20 hr, then removed and replaced with culture medium with serum. 24 hr later CdCl₂ was added to the cells to stimulate transcription of p53as mRNA from the plasmid DNA and enhance the expression of p53as protein. Two days later, cells were harvested by trypsinization, stained with anti-p53as antibody and analyzed by flow cytometry as described in the original patent application.

### Results of p53as expression studies

### Insect cells

Approximately 12% of insect cells infected with the baculovirus vector containing p53as expressed p53as antigen activity compared to 0% of insect cell controls lacking exogenous p53as cDNA. Insect cells expressing p53as protein were primarily in the G2 phase of the cell cycle or in a "tail" representing cells containing >G2 DNA content (Fig. 18A). Control insect cells were primarily distributed in the G1 phase of the cell cycle.

The significance of these studies is likely to be relevant to cell cycle control in mammalian cells. Other cells which do not have the p53 gene have proven very useful for studies of the role of p53 protein in cell cycle control. For example, while yeast does not contain the p53 gene, studies of p53 in yeast have been done to take advantage of the knowledge of cell cycle checkpoints and cell cycle regulatory proteins gained using the yeast model. The studies in yeast have been very informative, since p53 protein behaves in yeast cells in a manner consistent with its cell cycle role in mammalian cells (Nigro et at., 1992; Bischoff et al., 1992).

### Mammalian cells

As in the case of the insect cells, mouse carcinoma cells transfected with the plasmid containing p53as cDNA were preferentially distributed in the G2 phase of the cell cycle or in a "tail" representing cells containing >G2 DNA content (Fig. 9).

### Summary and Interpretations of p53as expression studies

These data indicate that expression of p53as by introduction into cells leads to accumulation of cells in the G2 phase of the cell cycle or exit from the cycle to a state in which DNA content is greater than G2 cells. A likely explanation for this is an arrest of cells within G2 and failure to undergo mitosis and proceed to the G1 or G0 phase of the cycle.

### Significance of p53as expression studies

These data are consistent with a checkpoint function of p53as at the G2/M phase of the cell cycle. Activity of p53as expression in causing cells to exit from the cell cycle would have useful applications in gene therapy of proliferative disorders such as cancer or psoriasis.

Because human and mouse p53 proteins form complexes in cells, the construct containing mouse p53as cDNA is claimed for the purposes of gene expression in mammalian cells and nonmammalian cells for research purposes, including human cells, and for gene therapy in humans. In addition, a purified plasmid construct containing the human p53as homologue of mouse p53as, defined by insertion of human intron 10 sequences into a sequence containing wt p53 DNA (as defined in original patent application serial no. 08/100,486) is claimed for research purposes in mammalian and nonmammalian cells, and for gene therapy in humans.

Table 3. shows reactivities of antibodies against p53 proteins. Mouse p53 has 390 amino acids; human p53 393 amino acids. All antibodies are mouse monoclonals commercially available from Oncogene Science, Cambridge MA, except ApAs rabbit polyclonal specific for p53as protein which was made in Dr. Kulesz-Martin's laboratory, RPCI. Sources: Oncogene Science Catalogue, p. 8, 1992; Vajtesek et al., J. of Immunolog. Methods 151:237-244, 1992, ^{a}Wade-Evans, A. and Jenkins, J. R. EMBO J., 4:699-706, 1985, ^{b}Gannon, EMSO, 9:1595-1602, 1990, ^{c}Stephen, C.W. and Lane, D.P., J. Mol. Biol., 5:577-583, 1992 and ^{d}Kulesz-Martin et al., Mol. Cell. Biol., in press, March 1994.

Table 4. shows p53 DNA Binding Sequences used for assay of p53as protein binding activity.

Table 5 shows Activation of 50-2 Muscle Creatinin Kinase Reporter Plasmid by p53as in Mouse Cells. Spontaneously arising immortalized murine BALB/c embryo fibroblast (10) 1 is a p53 negative cell line which was transfected with 2µg pSV-β-gal plasmid (Promega), 3µg 50-2 plasmid (Dr. Levine) which contains 2 copies of a 50 bp sequence corresponding to p53 binding site in muscle creatinine kinase promoter-enhancer upstream of a basic CAT reporter plasmid, and 5µg pCMVp53as, pCMVp53r or pCMV vector control. Transfection was done using the calcium phosphate method. The cells were harvested 48 hours post-transfection and lysed by three freeze-thaw cycles. The supernatant was assayed for CAT activity using diffusion.

Table 6 shows activation of PG₁₃CAT Plasmid by p53as in Human Saos-2 Cells. The human osteosarcoma Saos-2 cells were transfected with 3µg of PG13CAT plasmid, which contains 13 repeats of p53-binding site upstream of a CAT reporter gene with a basal promoter and 5µg pCMVp53as plasmid, pCMVp53r or pCMV vector control plasmid. Transfection was done using the calcium phosphate method and the total transfected DNA per plate was adjusted to 10µg with herring sperm DNA. The cells were harvested 48 hours post-transfection and lysed by three freeze-thaw cycles. Cell debris was removed by centrifugation and the supernatant was assayed for CAT activity using the diffusion method.

Tables 5 and 6 demonstrate that p53as has transcriptional activity. Plasmids containing p53as, or p53 as a control, were introduced into mouse or human cells along with a plasmid containing a p53 binding site upstream of a reporter gene. The reporter was activated by p53as, with p53 as a positive control, but not by the vector without p53as. By comparison, the background activity of the reporter plasmid alone was low. The reporter sequences 50-2 and PG13 are a promoter-enhancer sequence (Zambetti et al., Genes Dev. 6, 1143-1152, 1992) and a consensus p53 binding sequence (Kern et al., Science 256, 827-830, 1992). p53as was active on mouse (50-2) or human (PG13) p53 binding sequences, and in both mouse and human cells.

Table 7 shows Activation of the WAF-1 Promoter by p53as in Mouse Cells. Cells of mouse fibroblast line Dr. Levine (10)l were transfected with 2µg pSVCAT (Promega). 3µg WWP-1 (Dr. B. Vogelstein) which contains the WAF-1 promoter upstream of a luciferase reporter gene and 5µg of pCMVp53as, pCMVp53r or pCMV vector control were transfected using the calcium phosphate method. 48 hours later the cells were harvested and lysed in reporter lysis buffer (Promega). The supernatant was assayed for luciferase activity using a LB9501 luminometer. Luciferase activity was standardized to CAT activity.

Table 7 demonstrates that p53as activates transcription from an endogenous promoter region of a growth inhibitory gene, WAF-1, cipl, sdi, p21 (El-Deiry et al., Cell 75, 817-825, 1993; Gu et al., Nature 366, 707-710, 1993; Harper et al., Cell 75, 805-816, 1993; Xiong et al., Nature 366, 701-704, 1993; and Serranto et al., Nature 366, 704-705, 1993) recently reported to be a target gene of p53 in cells and a cell cycle control gene. The activity of p53as for the WAF-1 promoter is therefore strong evidence that p53as has transcriptional activity *in vivo* for genes functional in cell cycle control.

The similarities between p53 and p53as are that both act as tumor suppressors, both activate transcription and both form tetramers which bind specifically to DNA. The differences between p53as and p53 are that the p53 protein is activated under conditions in which p53 requires activation, p53as is associated with a different cell cycle stage compared to p53 and therefore have different functions or different regulation of activity. These differences suggest that plasmids containing p53as and the antibodies for p53as have utility as described herein and that utility may be different in some respects from that of plasmids containing p53.

For example, tumor cells which express different ratios of p53as and p53, as detected at the RNA level, or at the protein level by anti-p53as antibodies may have different characteristics or different sensitivity to anti-cancer treatments. Different characteristics identifiable by reactivity with p53as antibodies could aid in the diagnosis of cancer, prognosis for individual patients with tumors and decisions about treatment based on the competency of the p53as functions in the tumor. Antibodies to p53 are being used clinically in diagnosis of human cancers because expression of p53 at detectable levels has been found to correlate with cancer prognosis for a variety of human tumor types. It is known that tumors with defective p53 genes (more than 50% of all human cancers) fail to control cell cycle progression normally. Since p53 and p53as have different cell cycle associations, tumor typing using specific p53as antibodies and specific anti-p53 antibodies may increase the value of typing individual tumors according to their expression of tumor suppressor gene products such as p53. Such tumor typing may provide useful information in the diagnosis, prognosis, and treatment strategy of individual patient cancers.

Gene therapy strategies are being devised for introduction of anti-cancer genes into tumors for treatment of human cancers. Plasmids or vectors containing the p53as sequence of mouse or human may be useful for gene therapy approaches, based on the ability of plasmids containing mouse p53as to act as a tumor suppressor and to activate transcription of p53 target genes.

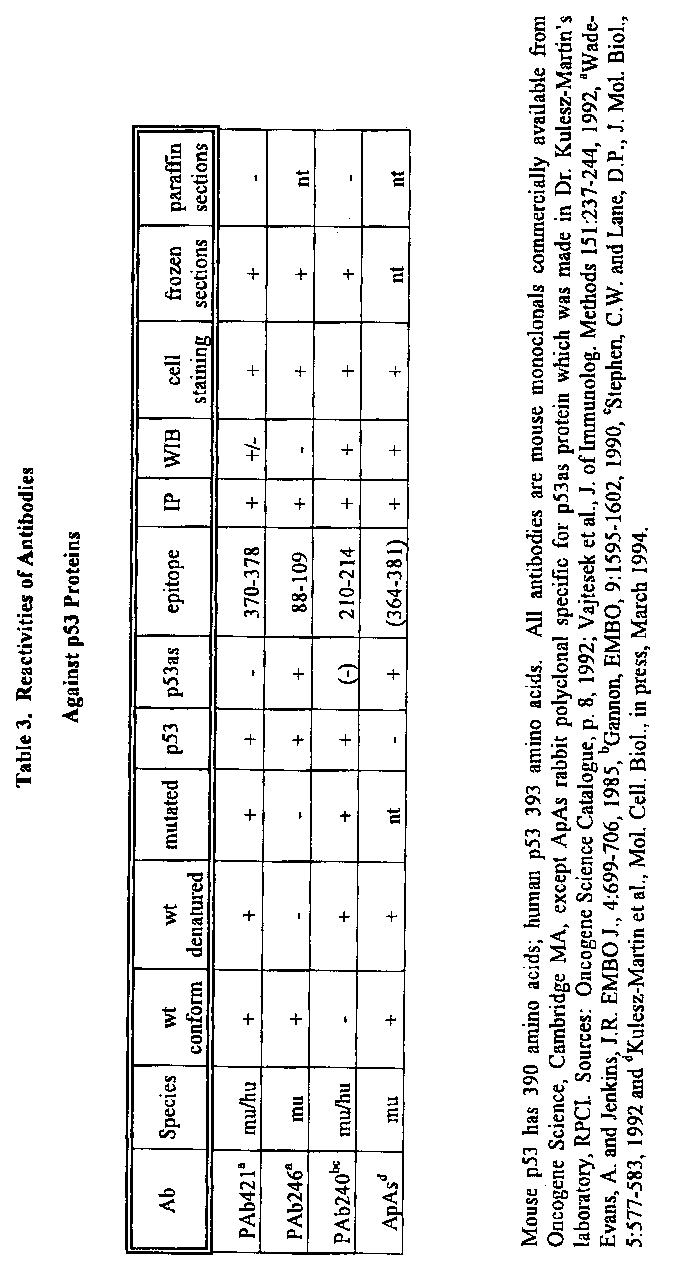

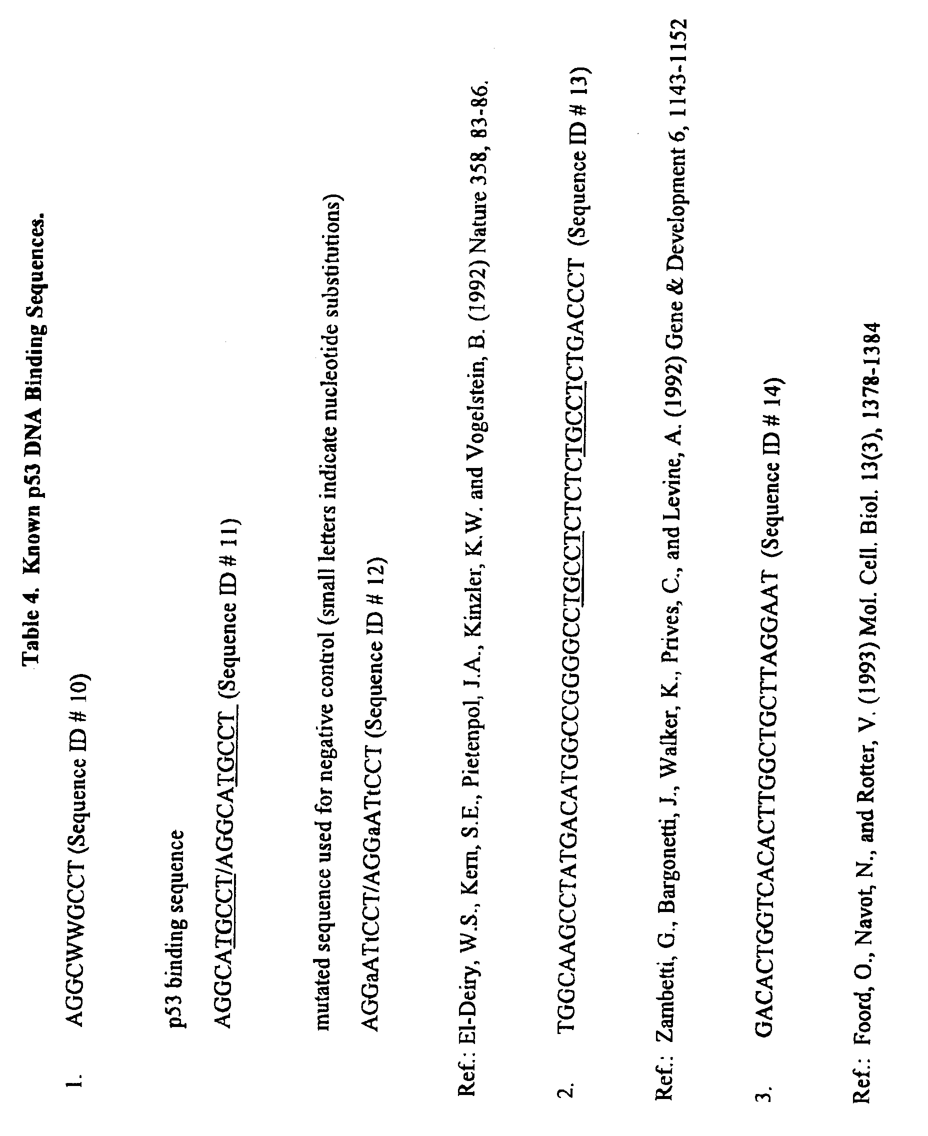

**Table 5**

| **Activation of 50-2 Muscle Creatinine Kinase Reporter Plasmid by p53as in Mouse Cells** | | |
|---|---|---|
| | CAT Activity (cpm) | Fold of activation |
| Vector | 1,457 | 1 |
| p53as | 37,185 | 26 |
| p53 | 39,855 | 27 |

**Table 6**

| **Activation of PG**_{**13**}**CAT Plasmid by p53as in Human Saos-2 Cells** | | |
|---|---|---|
| | CAT Activity (cpm) | Fold of activation |
| Vector | 2,539 | 1 |
| p53as | 23,319 | 9 |
| p53 | 35,190 | 14 |

**Table 7**

| **Activation of the WAF-1 Promoter by p53as in Mouse Cells** | | |
|---|---|---|
| | Luciferase Activity | Fold of activation |
| Vector | 481 | 1 |
| p53as | 6,855 | 14 |
| p53 | 7,010 | 15 |

## Claims

1. A synthetic p53as protein for a mammal having at least 80% identity with p53 protein from that mammal and which has a same sequence specific binding on the cellular level as active p53 protein for that mammal except that said p53as sequence specific binding remains active in cellular environments in which p53 sequence specific binding is deactivated, said p53as differing from p53 within the final 50 carboxy terminal amino acids of p53, p53as containing a specific antigen site not present in p53, which gives rise to an antibody unique for p53as.

2. An antibody which specifically binds to a synthetic mammalian p53as protein of Claim 1 and does not bind to a normal p53 from the same species wherein said antibody binds to an epitope present in a peptide unique to the p53as.

3. An antibody of Claim 2 specific for a synthetic protein designated p53as, which protein has at least 90% sequential correspondence with a known normal growth controlling protein p53 of a mammal, until the final 50 amino acids at the carboxy terminal end of the p53 protein, the final amino acids of p53as protein proximate the carboxy terminus being at least partly different than the carboxy terminus final 50 amino acids of the p53 protein, in that certain amino acids in the terminal sequence of p53as are different than amino acids in the final 50 amino acids in p53, p53as functioning as a growth regulator in a manner similar to active p53 within cellular environments where p53 loses activity due to loss of sequence specific binding.

4. The antibody of Claim 2 wherein the antibody is a polyclonal antibody.

5. The antibody of Claim 2 wherein the antibody is a monoclonal antibody.

6. The antibody of Claim 2 wherein the p53as carboxy terminal sequence is longer or shorter than the 50 amino acid terminal sequence of p53.

7. A method for use of an antibody of Claim 1 or Claim 2 for characterization of unknown cell products by reacting the unknown cell products with the antibody to determine the presence and concentrations of p53as and comparing the determined concentrations with concentrations in known cell products for assistance in characterizing of the unknown cell products, said antibody specifically binding to mammalian p53as protein and not binding to normal p53 protein from the same species, wherein said antibody binds to an epitope present in a peptide unique to the p53as.

8. A plasmid which generates the synthetic p53as protein of Claim 1.

9. A viral vector which generates the p53as protein of Claim 1.

10. A purified peptide designated p53as peptide, which peptide is present in the p53as protein of Claim 1 and is within a unique carboxy terminal region which distinguishes p53as protein from p53 protein.
